(19) Europäisches Patentamt
European Patent Office
Office européen des brevets

(11) **EP 3 091 454 B1**

(12) **EUROPÄISCHE PATENTSCHRIFT**

(45) Veröffentlichungstag und Bekanntmachung des Hinweises auf die Patenterteilung:
**02.10.2019 Patentblatt 2019/40**

(21) Anmeldenummer: **16152909.4**

(22) Anmeldetag: **10.11.2003**

(51) Int Cl.:
*G06F 17/50* (2006.01)      *G05B 19/4097* (2006.01)
*G06F 3/00* (2006.01)      *G06T 9/00* (2006.01)
*A61C 13/00* (2006.01)      *A61C 9/00* (2006.01)

(54) **VERFAHREN ZUR HERSTELLUNG VON ZAHNERSATZTEILEN ODER ZAHNRESTAURATIONEN UNTER VERWENDUNG ELEKTRONISCHER ZAHNDARSTELLUNGEN**

METHOD FOR PRODUCING DENTURE PARTS OR TOOTH RESTORATIONS USING ELECTRONIC DENTAL REPRESENTATIONS

PROCEDE DE FABRICATION DE PROTHESES DENTAIRES OU DE RESTAURATIONS DENTAIRES A L'AIDE DE REPRESENTATION ELECTRONIQUE DE LA DENT

(84) Benannte Vertragsstaaten:
**AT BE BG CH CY CZ DE DK EE ES FI FR GB GR HU IE IT LI LU MC NL PT RO SE SI SK TR**

(30) Priorität: **11.11.2002 DE 10252298**

(43) Veröffentlichungstag der Anmeldung:
**09.11.2016 Patentblatt 2016/45**

(62) Dokumentnummer(n) der früheren Anmeldung(en) nach Art. 76 EPÜ:
**03785641.6 / 1 581 896**

(73) Patentinhaber: **Sirona Dental Systems GmbH**
**64625 Bensheim (DE)**

(72) Erfinder: **Mehl, Albert**
**6340 Baar (CH)**

(74) Vertreter: **Özer, Alpdeniz et al**
**Dentsply Sirona**
**Legal IP Department**
**Fabrikstraße 31**
**64625 Bensheim (DE)**

(56) Entgegenhaltungen:
**EP-A1- 1 276 072      EP-A1- 1 304 088**
**US-A- 4 936 862      US-A- 5 027 281**
**US-A- 5 092 022      US-A- 5 257 203**
**US-A1- 2002 015 934**

- **SALIGER, G: "CAD/CAM in aesthetic dentistry; Designing a CEREC crown", QUENTESSENCE, CHICAGO, 1996 ED. W:H: MÖRMANN, 31. Dezember 1996 (1996-12-31), XP001180141,**
- **R. LUTHARDT P. KÜHMSTEDT P. BRAKHAGE: "Digitalisierung vollständiger Kiefermodelle und CAD-Modellation von Okklusalflächen", NEUE TECHNOLOGIE, 1. Januar 1999 (1999-01-01), XP001180082,**

**Beschreibung**

Die Erfindung betrifft ein Verfahren zur Herstellung von Zahnmodellen, Zahnersatzteilen oder Zahnrestaurationen reparaturbedürftiger Zähne bzw. Defektsituationen.

Für die Versorgung von Zahndefekten stehen verschiedene Möglichkeiten zur Verfügung. Eine Möglichkeit ist die direkte Applikation von Füllungsmaterial im Mund, d.h. der Zahnarzt entfernt die Karies und füllt das Loch in der gleichen Sitzung mit einem Füllungsmaterial. Diese Vorgehensweise wird vor allem bei kleineren Defekten gewählt. Für größere Defekte greift man eher auf Materialien wie Metall oder Keramiken etc. zurück, die nicht direkt im Mund angefertigt werden können. Auch ist die Kauflächengestaltung im Mund bei größeren Defekten eher problematisch und schwer durchzuführen. Daher wird beim Zahnarzt nach erfolgter Präparation des Zahnes eine Abformung durchgeführt. Diese Abformung wird in ein zahntechnisches Labor gegeben und ein Gipsmodell erstellt. Unter Berücksichtigung der Gegenbezahnung und eventuell unter Einbeziehung der Kieferbewegungen in Form von Artikulatoren kann dann die entsprechende Zahnrestauration oder Zahnersatzteil angefertigt werden. Dies können z.B. Inlays, Onlays, Teilkronen, Kronen, Brücken, Teleskopkronen, Teilprothesen etc. sein. Die Herstellung einer solchen Restauration ist allerdings sehr aufwendig. Nach der Abformung und Gipsmodellherstellung mit Zuordnung des Gegenkiefers erfolgt das Aufwachsen bzw. Sintern, Einbetten, Gießen oder Pressen, Ausarbeiten, Aufpassen und Polieren. Die hohe Anzahl der Schritte und die beschränkten technischen Möglichkeiten im zahntechnischen Labor führen dazu, dass zum einen sich Verarbeitungsfehler einschleichen können und die Materialqualität der fertigen Versorgung nicht optimal ist und zum anderen nicht alle Materialien gefertigt werden können (z.B. Hochleistungskeramiken). Zusätzlich führt der hohe Arbeitsaufwand auch zu hohen Kosten.

Als Alternative zur konventionellen Herstellungsweise wird inzwischen die CAD/CAM-Technologie gesehen, bei der mit Unterstützung von Computerverfahren die Anfertigung von Zahnrestaurationen und Zahnersatzteile durchgeführt wird. Vereinfacht gesprochen gliedert sich der Prozeß in:

1. dreidimensionaler Datenerfassung der Präparation bzw. mehrere Präparationen

2. Erzeugung eines CAD-Datensatzes der Zahnestauration, d.h. Konstruktion bzw Berechnung der Außenhülle und/oder interaktive Modellation der Außenhülle am Bildschirm

3. Bearbeitung des fertigen CAD-Datensatzes in einer computergesteuerten Fräs- bzw. Schleifmaschine (z.B. CNC) oder Rapid-Prototyping.Systemen.

Der Vorteil eines solchen Verfahrens liegt auf der Hand:

1. Kostenersparnis durch Automatisierung und damit Zeitersparnis

2. Verwendung industriell gefertigter Materialien. Diese können unter optimaleren Bedingungen als im Labor gesintert, gegossen etc. werden und weisen daher bessere Materialeigenschaften auf. Speziell für Keramiken und Titan sind diese Vorteile bereits eingehend untersucht worden.

3. Zahnersatz wird mit gleichbleibender Qualität produziert. Es kommt zu keinen Schwankungen durch Verarbeitungsfehler wie bei konventionellen Herstellungsprozessen.

4. Völlig neuartige Materialien wie Zirkonoxid-Keramiken etc, die bisher im konventionellen zahntechnischen Prozess nicht oder nur mit hohem Aufwand zu bearbeiten waren, lassen sich mit dem CNC-Verfahren anfertigen.

Einige Systeme sind bereits im Einsatz. Eine aktuelle Übersicht ist zum Beispiel der Ausgabe der ZWP (Dez. 2001, Mehl) zu entnehmen. Des weiteren werden in den Patentschriften US 5217375, EP 0643948, EP 0634150, EP 0913130 A2 und WO 0239056 solche Systeme bzw. einzelne Aspekte solcher Systeme beschrieben.

Ein Problem, das bisher nicht gelöst wurde, ist die möglichst automatisierte Herstellung von Zahnrestaurationen, die bereits eine Kaufläche aufweisen, die allen funktionellen und morphologischen Kriterien einer Kaufläche entspricht und optimal an die Gegenzahnsituation angepasst werden kann.

Bei den meisten Systemen ist zur Zeit nur die Herstellung von Gerüsten möglich. Ähnlich der konventionellen Vorgehensweise, bei der man z.B. ein Metallgerüst mit Keramik oder Kunststoff verblendet (gilt auch für andere Materialien wie spezielle Keramik- oder Kunststoffgerüste), wird das Grundgerüst im CAD/CAM-Prozess erstellt und anschließend zumindest Teile der Kaufläche und noch fehlende Außenflächen konventionell mit Keramik, Komposit etc. verblendet. Diese Gerüste können z.B. in der CAD-Software (Konstruktionssoftware) durch Vergrößerung der Präparation bzw. Berechnung einer Oberfläche, die in einem bestimmten, wählbaren Abstand (=Schichtstärke des Gerüstes) zur Präparationsoberfläche liegt, erreicht werden. Zusätzlich ist noch die Einbeziehung von "Ausbuchtungen" und "Deformationen" möglich. In EP 0913130 A2 ist mit Fig.13b solch ein Vorgehen zu sehen. Ein weiteres Beispiel ist in EP 06 43 948 A1 beschrieben.

Für die automatische Generierung einer Kaufläche, die nach allen gewünschten Kriterien und Anforderungen an eine gute Zahnrestauration bzw. Zahnersatzteil gestaltet ist, ist noch kein Verfahren vorhanden. Dies wäre jedoch von besonderer Bedeutung, da damit die Einsatzfähigkeit und die Kosteneffizienz eines CAD/CAM-Systems gesteigert und damit vor allem die CAD/CAM-Technologie überhaupt im großen Rahmen in der Zahnmedizin etabliert werden kann. Gleichzeitig muß mit diesem Verfahren auch die Möglichkeit bestehen, den berechneten Zahnersatz in einer Maschine anfertigen zu können.

Verschiedene Verfahren zur Kauflächengestaltung werden in der Literatur und in den Patentschriften beschrieben. Für die Rekonstruktion von Inlay-Flächen werden sowohl die Linearmethode als auch verschiedene Extrapolationsmethoden beschrieben (Mattiola, A., Mörmann, W.H. und Lutz, F.: "Computerunterstützte Okklusion von Cerec 2 Inlays und Overlays". Schweiz Monatsschr Zahnmed 105:1283-1290 (1995); Kunzelmann, K.-H., Mehl, A., Pelka, M.: Automatische Rekonstruktion von Kauflächen computergenerierter Restaurationen. Zahnärztl Welt/Rundschau 102, 695 - 703 (1993)). Bei der Linearmethode werden gegenüberliegende Punkte der Kavitätengrenze (meistens in oro-vestibulärer Richtung) einfach durch eine Gerade verbunden und so der Defekt aufgefüllt. Bei der Extrapolation wird die Steigung (Gradient) der noch vorhandenen Restzahnsubstanz in den Defekt hinein fortgesetzt und so die Oberfläche rekonstruiert. Es ist offensichtlich, das mit dieser Vorgehensweise nur angenähert etwas kauflächenähnliches entstehen kann. Die Einbeziehung morphologischer Kriterien und die Gegenbezahnungssituation ist nicht möglich. Gleichzeitig ist dieses Verfahren nur für relativ kleine Defekte geeignet.

Eine zweite Möglichkeit besteht in einer weiteren dreidimensionalen optischen Vermessung entweder der vorhandenen Kaufläche, bevor der Zahn beschliffen wird, oder einer individuell mit Wachs oder Kunststoff modellierten Kaufläche (z.B. Mattiola, A., Mörmann, W.H. und Lutz, F.: "Computerunterstützte Okklusion von Cerec 2 Inlays und Overlays". Schweiz Monatsschr Zahnmed 105:1283-1290 (1995), Mehl, A., Gloger, W., Hickel, R.: Erzeugung von CAD-Datensätzen für Inlays und Kronen mit funktionellen Kauflächen. Dtsch Zahnärztl Z 52, 520-524 (1997)). Durch Anklicken oder Auswahl von Referenzpunkten an den Nachbarzähnen kann die Präparations- und die Kauflächenmessung zueinander positioniert und die vollständige Restauration ergänzt werden. Hier muß allerdings eine Wachsmodellation manuell erstellt werden, so dass der Vorteil der Automatisierung durch das CAD/CAM-System nicht mehr gegeben ist. In den meisten Fällen bei der Versorgung eines Zahnes wird die Ausgangskaufläche aufgrund vorhandener kariöser Defekte oder insuffizienter Versorgungen nicht verwendbar sein, so dass auch diese Möglichkeit nur für einen kleinen begrenzten Indikationsbereich beschränkt bleibt. Eine zusätzliche Möglichkeit wird in WO 0239056 vorgestellt. Hier wird ein Patientenarchivierungssystem z.B. Chipkarte für den Patienten, beschrieben, das beinhaltet, das Zahndaten gespeichert werden. Diese Zahndaten können dann zu einem späteren Zeitpunkt, wenn beim Patienten Versorgungen angefertigt werden, wieder verwendet werden und für die Rekonstruktion des Defektes dienen. Hier wäre jedenfalls gewährleistet, dass die ergänzte Kaufläche morphologisch und funktionell optimal dem gnathologischen System angepaßt ist. Allerdings muss man bei diesen Verfahren mit entsprechenden Vorlaufszeiten rechnen, so dass vorerst für die Versorgungen einer breiten Masse andere Verfahren herangezogen werden müssen.

Weitere Möglichkeiten im Zusammenhang mit der Einbeziehung von Kauflächengeometrien in den CAD/CAM-Prozess werden in den folgenden Erfindungen beschrieben. Aus DE 198 38 239 A1 sind Gruppen von Rohlingen für Dentalrestaurationen bekannt, die unterschiedlichen Zahntypen zugeordnet werden und deren Außengeometrien aus Mittelwerten, die einschlägigen Lehrbüchern entnommen werden, für den jeweiligen Zahntyp bestimmt werden. Dabei handelt es sich aber nicht um eine mathematische, für die CAD/CAM-Rekonstruktion von Zahnrestaurationen verwendbare Beschreibung von Zahnoberflächen, sondern nur um eine ungefähre Maximum -Minimum Abschätzung für die groben Außenmaße von Rohlingen, aus denen dann die gewünschte individuelle Zahnrestauration herausgefräst werden soll. Die Mittelwerte, die aus der Literatur übernommen werden können, sind außerdem nur Längen-, Breiten- oder ähnliche lineare Messungen, die in keiner Weise auch nur annähernd eine Kaufläche für den computergestützten Rekonstruktionsprozess beschreiben können.

Aus DE 199 23 978 A1 ist ein Verfahren zur computergestützten patientenspezifischen Darstellung und Planung zahnärztlicher und/oder zahnprothetischer Arbeiten bekannt, bei denen eine digitalisierte Bilddatenbank mit einer Vielzahl von Modellzahn- und Kieferansichten erstellt wird, wobei die Modellansichten gesunde und dentale Krankheitsbefunde aufweisende Objekte, z.B. Einzelzähne umfassen, wobei die Bilddatenbank Abbildungen typischer Mundregionen beinhaltet. Dieses Verfahren dient als computergestütztes Expertensystem für die Therapieplanung und -entscheidung von zahnärztlichen Behandlungen. Für die dreidimensionale Rekonstruktion von Zahndefekten, wie es im CAD/CAM-Prozess zur Herstellung von Zahnersatz notwendig ist, ist dieses Verfahren nicht geeignet, da patientenspezifische Befunddaten nicht für die exakte individuelle Anpassung der Bilddatenbanken ausreichen. Die Bilddatenbank soll auch nur typische Standardformen für das Planungsgespräch mit dem Patienten zur Verfügung stellen, eine Veränderung durch Kombinationen dieser Daten zu einem neuen repräsentativen Datensatz wird nicht vorgenommen.

Aus EP 06 43 948 A1 ist ein Verfahren zur Herstellung einer Dentalrestauration bekannt, bei dem eine selbstlernende Datenbibliothek von Zahnbasisformen zum Einsatz kommt. Die Vorgehensweise beschränkt sich dabei nur auf die Erstellung von Kronengerüsten und beinhaltet das Lernen nur solcher Parameter wie Schichtdicke, Lokalisierung und Dicke von Wölbungen, ungefährer Verlauf der Präparationslinie. Dieses einfache "Lernen" führt nicht zu mathematischen oder parametrischen Beschreibungen von Zahnoberflächen, die für die Rekonstruktion von individuellen Zahndefekten mit kompletter Außengeometrie wie anatomisch und funktionell geformte Inlays, Onlays, Kronen und Brücken geeignet sind. Insbesondere kann hiermit auch keine Berücksichtigung der umgebenden Restgebisssituation wie Nachbarzähne und Gegenbezahnung stattfinden. Weiterhin wird hier nicht die von der Natur vorgegebene Form nachgebildet, sondern nur die auf der Erfahrung des/der Zahntechniker / Experten beruhenden physikalischen Konstruktionsparameter von Gerüsten ermittelt.

Aus US 52 57 203 ist ein Verfahren zur Herstellung einer Dentalrestauration bekannt, bei dem eine Datenbank standardisierter generischer Zahnformen eingesetzt wird, wobei diese generischen Zahnformen typischerweise computerbasierte Darstellungen von standardisierten Gipsmodellen von Zähnen sind. Bei den in diesem Verfahren benutzten generischen Zahnformen handelt es sich nicht um rechnerisch oder algorithmisch aus einer Datenbank abgeleitete Zahnformen und damit nicht um im Sinne der vorliegenden Erfindung beschriebene generische Zahnmodelle, sondern um standardisierte Gipsmodelle, die nur dreidimensional gescannt werden und dieser Datensatz für die Rekonstruktion verwendet wird. Der Nachteil ist auch hier, dass der Standardisierung kein allgemeingültiges Konstruktionsprinzip zu Grunde liegt und die Gestaltung nur auf der manuellen Geschicklichkeit und der Erfahrung einzelner Experten mit entsprechender Einschränkung der in der Natur vorkommenden Formvielfalt beruht.

Stand der Technik

Aus der US 2002/0015934 A1 ist eine Zahndatenbank bekannt, die patientenspezifische Merkmale einer Vielzahl von Patienten berücksichtigt. Dabei kann ein virtueller Modellzahn verändert werden.

Eine weitere Möglichkeit zur Herstellung von Zahnrestaurationen wird in "Saliger, G., Designing a CEREC crown. In Cerec 10 year anniversary Smposium, ed. W.H. Mörmann. Quintessence, Chicago, 1996" oder DE 19642247 beschrieben. Hier wird der Datensatz eines Modellzahnes an den präparierten Zahn angepasst und adaptiert. Im wesentlichen wird dieser Modellzahn entsprechend der mesial-distalen Ausdehnung des Defektes skaliert, translatiert und rotiert. Eine elastische Deformation kann das Ergebnis noch verbessern.

Bei Saliger 1996 (s.o) wird eine anschließende interaktive Möglichkeit der Rotation und der Kontrolle der Kaufläche zum Gegenzahn bereitgestellt. Zusätzlich können die Höcker in ihrer Position verändert werden. Dies erfolgt alles interaktiv. Zum Schluß wird dann die Zahnrestauration gefräst.

Das Problem bei all den erwähnten Vorgehensweisen liegt vor allem in folgenden Fakten begründet:

-    Kontaktpunkte zum Gegenzahn werden erst im nachhinein festgelegt, indem durch interaktive Verzerrungen die Anpassung erfolgt oder der Modellzahn solange verändert wird, bis Berührungen mit der Hülle vorliegen. Dies führt oft zu völlig zahnuntypischen Formen, da der Modellzahn von Anfang an nicht optimal in die Gesamtsituation angepasst wird.

-    Es gibt keine automatisiertes Vorgehen für die Auswahl eines besten Modellzahnes (falls mehrere zur Auswahl stehen). Bis jetzt erfolgt das nur auf Grund visueller Regeln.

-    Die Arbeit und interaktive Veränderung am Monitor ist in ihrer Auswirkung im dreidimensionalen nur schwer vorstellbar und daher für einen mit der Computer-Arbeit weniger Erfahrenen nur nach langer Einübungszeit und täglicher Routine zu beherrschen.

-    Die Morphologie der Nachbarzähne bzw. Antagonisten oder auch der entsprechende im gleichen Kiefer gegenüberliegende Zahntyp wird nicht herangezogen. Dies ist in manchen Fällen wichtig, um eine harmonische Eingliederung der Restauration in das Kiefersystem zu gewährleisten.

-    Veränderungen des Modellzahnes durch Skalierung, Höckerpositionierung und interaktiven Deformationen müssen nicht unbedingt zu zahnähnlichen Oberflächen führen.

-    Für alle interaktiven oder automatischen Anpassungen gibt es bis jetzt kein Verfahren, dass garantiert, dass nach Veränderung wieder eine einem natürlichen Zahn sehr ähnliche Kaufläche entsteht. Nachdem bis heute die Kriterien einer guten funktionell und statisch gestalteten Kaufläche wissenschaftlich noch nicht bekannt und auch nachgewiesen sind, muss die Forderung für jede Restauration lauten, dass sie möglichst nahe an natürliche Gegebenheiten und Formen angenähert wird, um so keine Langzeitschäden an den Zähnen, im Gewebe und im Gelenk zu verursachen.

Eine Überwindung vorausgehend angesprochener Probleme ist mit der vorliegenden Erfindung gelungen, welche die Herstellung von Zahnrestaurationen, Zahnersatzteilen oder Zahnmodellen mit Kauflächen und/oder Oberflächen, die einem natürlichen Zahn sehr nahe kommen und sich funktionell und morphologisch optimal in die Kiefersituation einfügen, ermöglicht, wobei der Herstellungsvorgang mehr automatisiert werden kann, d.h. mit wesentlich weniger Interaktionen und fehlerfreier, d.h. benutzerfreundlicher, ablaufen kann.

Unter Zahnersatzteile versteht man in dieser Patentschrift Teile oder die Gesamtheit von Total- oder Teilprothesen (z.B. Teleskopprothese, Klammerprothese, Interimsprothesen etc.) oder auch Implantataufbauten, unter Zahnrestaurationen Brücken, Teleskopkronen (Primär- und Sekundärteile), Kronen, Inlays, Onlays, Overlays und Teilkronen. Zahnmodelle werden verwendet als Prothesenzähne, als eigenständige Modelle, als Bestandteile von Übungs-, Ausbildungs- und Anschauungsmodellen oder zur Darstellung in elektronischen Medien oder Printmedien. Abzugrenzen ist davon der Begriff des generischen Zahnmodells oder generischen Zahnmodelldatensatzes, wie er im Folgenden erklärt wird.

Die Erfindung schafft ein Verfahren zur Herstellung eines elektronischen Datensatzes eines generischen Zahnmodells unter Verwendung eines elektronischen Datensatzes eines für die Anfertigung eines Zahnersatzteils einer Zahnrestauration oder eines Zahnmodells verwendbaren Durchschnittszahns, wie es in Anspruch 1 angegeben ist. Des weiteren schafft die Erfindung Verfahren zur Herstellung von Zahnersatzteilen oder Zahnrestaurationen, wie sie in den Ansprüchen 5 9, und 16 angegeben sind. Eine Verwendung des Verfahrens zur Herstellung eines dreidimensionalen elektronischen Bildes des Durchschnittszahns bzw. des Verfahrens zur Herstellung eines elektronischen Datensatzes des generischen Zahnmodells ist in Anspruch 4 angegeben. Weiterbildungen der erfindungsgemäßen Verfahren sind in den abhängigen Ansprüchen angegeben. In Anspruch 18 wird eine Vorrichtung zur Visualisierung, Anpassung und Justierung eines generischen Zahnmodelldatensatzes angegeben.

Ein auf erfindungsgemäße Weise erhaltenes elektronisches Bild eines Durchschnittszahns oder der Datensatz eines generischen Zahnmodells eignet sich besonders gut als Ausgangsbasis für die Herstellung eines Zahnersatzteils, einer Zahnrestauration oder eines Zahnmodells, weil der Durchschnittszahn oder noch allgemeiner der generische Zahnmodelldatensatz im Gegensatz zu einem herkömmlichen elektronischen Zahnmodell nicht von mehr oder weniger mit der Natur übereinstimmenden Vorstellungen des Autors des elektronischen Zahnmodells basiert, sondern von reellen Zähnen bestimmt ist.

Wenn man beispielsweise eine Restauration für einen reparaturbedürftigen Zahn unter Zuhilfenahme eines erfindungsgemäß erhaltenen elektronischen Durchschnittszahns bzw. generischen Zahnmodells herstellt, gibt die in dem Durchschnittszahn bzw. generischen Zahnmodells zum Ausdruck kommende natürliche Zahnform den Ausschlag und nicht ein menschlicher Vorstellung entsprungenes Zahnmodell.

Bei der Herstellung beispielsweise einer Zahnrestauration kann man den erfindungsgemäß erhaltenen Durchschnittsdatensatz bzw. das generische Zahnmodell als Ausgangsbasis nehmen und diese Datensätze unter Anpassung an bestehen gebliebene Zahnflächenteile des reparaturbedürftigen Zahns oder der Restgebisssituation an den jeweils zu reparierenden Zahn anpassen, indem per interaktiven Eingriff oder durch softwaregesteuerte Automatik diese Datensätze abgewandelt werden, um die genannte Anpassung an die Zahnflächenreste des zu reparierenden Zahns oder der den zu reparierenden Zahn umgebenden Restgebisssituation vorzunehmen.

Besonders gute Ausgangsbedingungen erhält man für den generischen Zahnmodelldatensatz. Daher sehen das Verfahren gemäß Anspruch 1 vor, dass auf der Grundlage einer Korrespondenzanalyse eine Hauptachsenanalyse und eine Linearkombination in der in diesen Ansprüchen beschriebenen Weise durchgeführt werden, aus denen ein generischer Zahnmodelldatensatz erstellt wird. Mit Hilfe des generischen Zahnmodells lässt sich der Rahmen fest legen, innerhalb welchem eine Anpassung des Modelldatensatzes an das elektronische Bild der Reststruktur des zu reparierenden Zahns möglich ist, ohne sich aus dem Formenvorrat natürlicher Zahnformen weg zu bewegen. Die Anpassung des generischen Zahnmodelldatensatz an den zu reparierenden Teil des reparaturbedürftigen Zahns kann auf interaktive Weise oder auch vollautomatisch mittels Softwaresteuerung und -verarbeitung geschehen. Steuert man eine numerisch gesteuerte Maschine entsprechend einem auf diese Weise erhaltenen Datensatz, kommt man zu einem physischen Zahnteil, welches dem Aussehen der ehemaligen unversehrten Zahnfläche des zu reparierenden Zahns besonders gut nahe kommt, wobei man dieses Ergebnis auch auf für den Zahnmediziner oder Zahntechniker vergleichsweise einfache Weise erreichen kann.

Das Verfahren nach den Patentanspruch 1 befasst sich mit der Schaffung einer oder zumindest sehr weniger generischer Zahnmodelldatensätze eines bestimmten Zahntyps (z.B. OK-6er, oder auch großer, mittlerer und kleiner OK-6er, etc.). Diese Oberflächen erlauben schon für viele Situationen eine ausreichende zahnähnliche Rekonstruktion. Weiterhin ermöglicht der generische Zahnmodelldatensatz, das jede Veränderung, die unter gewissen Kriterien (siehe unten) an dieser Oberfläche durchgeführt wird, mit hoher Wahrscheinlichkeit wiederum eine natürliche Kaufläche ergibt und dass alle möglichen zugelassenen Varianten an Veränderungen die Gesamtheit nahezu aller in der Natur vorkommenden Zahnmorphologien beschreibt. Die Anzahl der Anpassungsvariablen ist dabei gering und die Rekonstruktion von Zahnoberflächen kann automatisiert werden.

Die Erzeugung dieses generischen Zahnmodelldatensatzes erfolgt dabei über eine möglichst große Anzahl von Datensätzen desselben Zahntyps. Im allgemeinen können die elektronischen Datensätze sowohl zwei- als auch dreidimensional vermessen sein. Zweidimensionale Vermessung geht z.B. mit der metrischen Photographie, dreidimensional z.B. mit Weißlicht-Streifenprojektion etc., auch Stereophotogrammetrische Verfahren wären denkbar. Zur Rekonstruktion von reparaturbedürftigen Zähnen und Defektsituationen benötigt man jedoch zumindest dreidimensional vermessene Datensätze. Beim Zahntyp kann es sich zum Beispiel um Molaren, Prämolaren, Eckzähne und Frontzähne handeln. Als Zahntyp kann aber auch der Oberkiefer(OK)-6er, Unterkiefer(UK)-4er, OK-1er etc. stehen. Möglich ist des weiteren eine Unterscheidung nach Alter und Abrasion, nach Geschlecht, nach Volkszugehörigkeit, nach Größe der Zähne, nach morphologischen Besonderheiten etc, z.B. können die Gruppen OK-7er im Alter von 50-60 Jahren, OK-6er mit und ohne Tuberculum Carabelli, UK-3er bei weiblichen Personen, Unterteilung in große, mittlere, kleine 6er etc. ein Beispiel für einen Zahntyp darstellen. Auch können z.B. benachbarte Zähne zu einem (kombinierten) Zahntyp zusammengefasst werden, um Zusammenhänge von Nachbarzähnen zu integrieren oder zu analysieren. Durch die Information des Nachbarzahnes könnte z.B. die Auswahl der Zahnoberfläche für den reparaturbedürftigen Zahn oder für die Defektsituation erfolgen. Der Begriff Zahntyp umfasst also eine je nach Aufgabenstellung sehr variable Gruppierungsmöglichkeit, was bei den Patentansprüchen in ihrer Allgemeinheit zu beachten ist.

Für einen bestimmten Zahntyp müssen die jeweiligen Datensätze in einem ersten Schritt zueinander referenziert werden (ins gleiche Koordinatensystem gebracht und ungefähr gleichmäßig ausgerichtet werden) und zwischen den Oberflächenpunkten des einen Datensatzes mit den jeweils anderen Datensätzen Korrespondenzen aufgebaut werden. Diese Korrespondenzen erfolgen z.B. zwischen markanten Punkten und Strukturen der Oberfläche. Diese Zuordnung kann manuell erfolgen, kann durch Suchen und Zuordnung von bestimmten charakteristischen Merkmalesstrukturen (Höckerform, Fissurenverlauf, Randleiste etc.) erfolgen. Wahlweise ist hier ein Prozeß vorzuziehen, der diese Korrespondenzpunkte und/oder -strukturen automatisch findet, da bis jetzt noch kein bewiesener metrisch erfaßbarer Sachverhalt vorliegt, welche wirklich die markanten Punkte bzw. Strukturen oder Eigenschaften eines bestimmten Zahntyps sind. Im Gegenteil, es gibt bis jetzt in der gesamten zahnmedizinischen Fachliteratur keinen Hinweis auf eine nur annähernde mathematische Beschreibung von Zahnoberflächen, die in irgendeiner Weise für den CAD/CAM-Prozess geeignet wäre.

Als eine mögliche Implementierungsvariante hat sich folgendes Vorgehen als machbar erwiesen: Zuerst werden die Datensätze der vermessenen Zahnoberflächen eines bestimmten Zahntyps ins gleiche Koordinatensystem gebracht, um eine möglichst gute Ausgangsbasis für die automatische Korrespondenzpunktbestimmung zu bekommen. Dies kann mit Matching-Routinen durch Minimierung der Abstandsfehlerfunktion erfolgen, wobei Rotations- und Translationsparameter ermittelt werden. Nach erfolgter Koordinatentransformation findet die Korrespondenzanalyse statt. Aus der Bildverarbeitung können hier modifizierte Algorithmen zum optischen Fluß mit Erfolg angewandt werden. Weiterhin kann durch elastisches Registrieren bzw. Matchen von bestimmten Merkmalen (Fissuren, Höckerspitzen, Höckerabhängen, Randleisten) zwischen den einzelnen Zahnoberflächen Korrespondenzen gebildet und Abbildungsvorschriften gefunden werden. Am Schluß erhält man die Zuordnung vieler Punkte durch Korrespondenzen zwischen allen Datensätzen.

Genauer wird dies im Folgenden anhand des Verfahrens des optischen Flusses spezifiziert. Ausgangsbasis sind m Bibliothekszahnflächen eines bestimmten Zahntyps, entnommen einer Zahnbibliothek, in der Form $z_j(x,y)$, mit j = 1,....m als Scandaten. Genauso erlaubt sind parametrische Darstellungen $z_j(u,v)$, mit $u = u(x,y)$, $v = v(x,y)$. Dies können z.B. Polarkoordinaten etc. sein. Beliebige komplizierte dreidimensionale Oberflächen mit Unterschneidungen können stückweise durch obige Funktionen angenähert werden. Im erweiterten Sinne sind für andere Verfahren auch beliebig dimensionale Beschreibungen von Zähnen erlaubt.

Ausgehend von einem Referenzzahn $z_R(x,y)$, mit $R \in \{1,...,m\}$, wird mittels einer Korrespondenzanalyse zu jedem Punkt des Referenzzahnes der korrespondierende Punkt auf der Kaufläche $z_j(x,y)$ gesucht. Dies kann auch durch hintereinander geschaltetes Verknüpfen von Korrespondenzen erfolgen, indem von einem Zahn ausgehend die Korrespondenz zu einem weiteren Zahn aufgebaut wird, von diesem neuen Zahn eine weitere Korrespondenz zu einem dritten Zahn etc. Zusätzlich kann vor jeder neuen Korrespondenzermittlung auch ein neuer Durchschnittszahn aus den vorhanden Korrespondenzen berechnet und als Ausgangsbasis für die neue Korrespondenzanalyse dienen. Insgesamt kann dies durch einen Algorithmus erreicht werden, der diese Korrespondenzen automatisch ohne Vorkenntnisse auffindet, wie in Patentanspruch 6 spezifiziert. Eine Möglichkeit ist das Verfahren des optischen Flusses (für beliebige 3D-Objekte sind auch andere Möglichkeiten beschrieben in Shelton, C.R.: 3D Correspondence. Master Thesis, Massachusetts Institute of Technologie, 1998). Als Ergebnis erhält man ein zu jedem Zahn $z_j(x,y)$ gehöriges zweidimensionales Vektorfeld $\vec{v}_j(x,y)$ mit:

$$\vec{v}_j(x,y) = \begin{pmatrix} \Delta x_j(x,y) \\ \Delta y_j(x,y) \end{pmatrix}$$

so daß zu jedem Koordinatenpaar (x,y) des Referenzzahnes $z_R(x,y)$ sich der korrespondierende Punkt des Zahnes $z_j(x'y')$ aus der Beziehung:

$$z_j(x + \Delta x_j(x, y), y + \Delta y_j(x, y))$$

ergibt. Bei Zahnoberflächen ist es sinnvoll, neben der Glattheit des Verschiebungsfeldes bezüglich der z-Koordinaten, auch die Glattheit bezüglich der Steigungen zu fordern, da Steigungen auch ein wesentliches Merkmal der Kauflächen darstellen. Auch kann man bei der Vorgehensweise der Korrespondenzanalyse versuchen, nach jeder neuen Korrespondenzfindung diesen Datensatz zu den schon korrespondierenden Datensätzen hinzuzufügen und daraus eine neue Linearkombination suchen, die den nächsten Datensatz, der noch nicht korrespondiert, möglichst gut annähert. Diese neue Linearkombination kann dann zur automatischen Korrespondenzfindung herangezogen werden. So können iterativ alle Datensätze in Korrespondenz gebracht werden.

Nachdem nicht alle Punkte einer Oberfläche eindeutig den Punkten der anderen Oberfläche zugeordnet werden können, kann man fordern, dass sich das Verschiebungsfeld bildlich wie eine elastische Membran verhält: Zwischen den eindeutigen Korrespondenzen ist diese Membran nahezu nicht verschieblich, während sie dazwischen, d.h. in den Bereichen mit unklaren oder schwachen Korrespondenzen, sich nahezu frei entspannen kann. Berechnet werden kann dies zum Beispiel durch Minimierung einer Energiefunktion, die aus einer Kopplung von vielen Federn zwischen den einzelnen Oberflächenpunkten besteht (Näherung für die kontinuierliche elastische Membran).

Eine interessante Erweiterung für die Berechnung des optischen Flusses besteht darin, dass man neben der 3D-Datenrepräsentation $z(x,y)$ weitere Kriterien oder Oberflächenbeschreibungen für die Korrespondenzanalyse heranzieht, wie in Patentanspruch 7 ausgewiesen. Dies könnte zum Beispiel das Gradientenfeld (Steigungen) der Zahnoberfläche sein. Gradienten beschreiben bestimmte Merkmale wie Kanten, Ecken oder stärkere Änderungen auf der Oberfläche besser als die reinen Höhendaten. Indem man nun einen neuen Merkmalsvektor $\vec{m}$ mit

$$\vec{m} = \begin{pmatrix} z(x, y) \\ \nabla z(x, y) \end{pmatrix}$$

bildet und eine neue Norm für diesen Merkmalsraum einführt:

$$\left\| \vec{m} \right\|^2 = z^2(x, y) + \beta \cdot (\nabla z(x, y))^2$$

wobei $\beta$ die Gewichtung des Gradientenfeldes im Verhältnis zum Höhenbild festlegt, so kann das Verschiebungsfeld $\vec{v}(x,y) = (\Delta x(x, y), \Delta y(x, y))^T$ für den Merkmalsvektor $\vec{m}$ analog zu oben berechnet werden, wenn man die Normen $\|\vec{m}_x\|^2$ und $\|\vec{m}_y\|^2$ und die jeweiligen Skalarprodukte $\langle \vec{m}_x, \vec{m}_y \rangle$ bzw. $\langle \vec{m}_x, \Delta \vec{m} \rangle$ verwendet. Natürlich könnte man sich auch mehrdimensionale Merkmalsvektoren vorstellen, indem man noch weitere Eigenschaften der Zahnoberfläche mit berücksichtigt. Dies könnten zum Beispiel Texturwerte, Krümmungen etc. sein. Der Gewichtungsfaktor $\beta$ (oder weitere Gewichtungsfaktoren) erlaubt die Möglichkeit, den jeweiligen Einfluss der einzelnen Merkmalsfelder festzulegen. Mit all diesen Maßnahmen liegt ein starkes Werkzeug vor, dass für die Zahnoberflächen eine automatische, ohne Vorkenntnisse erfordernde Analyse von Korrespondenzen ermöglicht.

Sind diese Zuordnungen gefunden, kann in einem nächsten Schritt der Referenzzahn als Vektor in einem 3n-dimensionalen Raum repräsentiert werden (n ist dabei die Anzahl der ausgewählten auf der Zahnoberfläche liegenden Punkte, idealerweise wird man ein äquidistantes Gitter zugrundelegen, die typische Anzahl von Punkten kann von 10000-200000 gehen):

$$\vec{D}_R = (x_1, y_1, z_R(x_1, y_1), x_2, y_2, z_R(x_2, y_2), \ldots, x_n, y_n, z_R(x_n, y_n))$$

In konsistenter Weise können dann ausgehend vom Referenzzahn (oder der Linearkombination) und dem entsprechenden Vektorfeld $\vec{v}_j(x,y)$ alle anderen Zähne der Bibliothek als 3n-dimensionaler Vektor dargestellt werden:

$$\vec{D}_j = (x_1 + \Delta x_j(x_1, y_1),\ y_1 + \Delta y_j(x_1, y_1),\ z_j(x_1 + \Delta x_j(x_1, y_1), y_1 + \Delta y_j(x_1, y_1)),$$
$$x_2 + \Delta x_j(x_2, y_2),\ y_2 + \Delta y_j(x_2, y_2),\ z_j(x_2 + \Delta x_j(x_2, y_2), y_2 + \Delta y_j(x_2, y_2)),.....,$$
$$x_n + \Delta x_j(x_n, y_n),\ y_n + \Delta y_j(x_n, y_n),\ z_j(x_n + \Delta x_j(x_n, y_n), y_n + \Delta y_j(x_n, y_n)))$$

Auf diese Weise repräsentieren gleiche Vektorkoordinaten, d.h. Indizes, auch jeweils korrespondierende Punkte und zwar zwischen allen Zähnen. Die Gesamtheit der m Vektoren, die den m Bibliothekszähnen entsprechen, spannen einen Raum auf, den man als den Zahnraum für den entsprechenden Zahntyp bezeichnet. Damit läßt sich nun auch der Durchschnittszahn $\vec{D}$ aus den einzelnen umgeformten Bibliothekszähnen $\vec{D}_j$ berechnen:

$$\vec{D} = \frac{1}{m} \cdot \sum_{j=1}^{m} \vec{D}_j$$

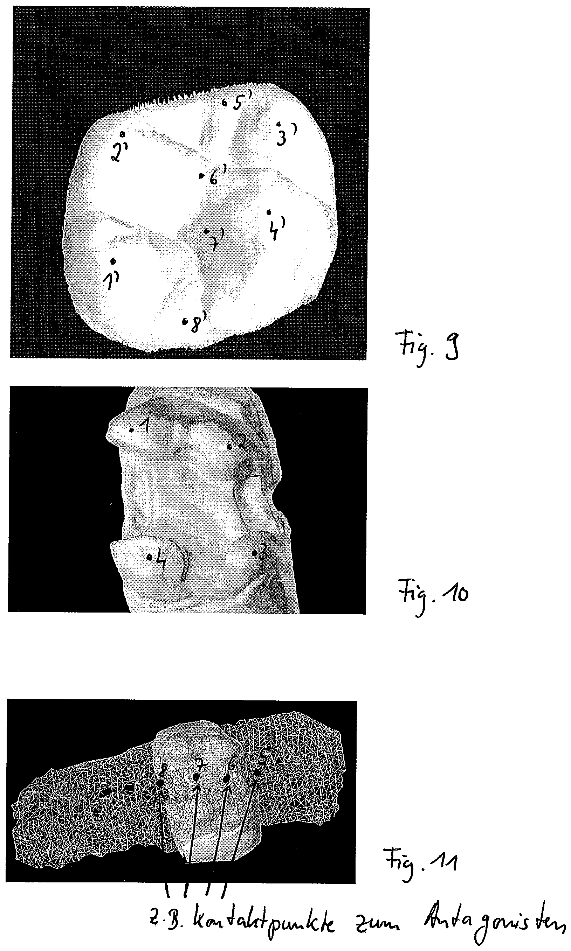

Man kann an dieser Stelle den neuen Durchschnittszahn wiederum als Referenzzahn verwenden und obigen Prozeß nochmals neu starten und auch öfters wiederholen. Damit kann der Durchschnittszahn noch allgemeiner bestimmt werden. Oder man nimmt verschiedene Referenzzähne und mittelt nachher das Ergebnis. Im Patentanspruch 1 wird dieser Durchschnittsdatensatz als Durchschnittszahn einer bestimmten Zahngruppe (Zahntyp) zur Verfügung gestellt (Fig. 9).

Liegen die einzelnen Zahnoberflächen als Vektoren vor, ist es mit hoher Wahrscheinlichkeit möglich, jeden neu hinzugekommenen Zahn $\vec{Z}$ als Linearkombination der vorhandene Zähne zu repräsentieren:

$$\vec{Z} \approx \sum_{j=1}^{m} \beta_j \cdot \vec{D}_j$$

Um die Anzahl der Linearfaktoren $\beta_j$ und der Zähne $\vec{D}_j$ zu reduzieren, bietet sich die Hauptachsenanalyse an. Nachdem jeder Zahntyp für den Experten durch bestimmte Merkmale erkennbar ist, sollten durch die Hauptachsentransformation auch diejenigen Komponenten großen Einfluss haben, die bestimmte Merkmale des Zahntyps charakterisieren. So erhält man durch die Linearkombination eines Teils der Hauptachsen eine ausreichende Beschreibung der meisten Zahnoberflächen.

Diese Hauptachsenanalyse kann auf den Zahndaten $\vec{D}_j$ direkt durchgeführt werden. Der verwendete Anteil p der sich ergebenden Hauptachsen (in der Regel die, die am meisten zur Varianz beitragen) wird durch Linearkombination (Linearfaktoren $a_i$ und Hauptkomponenten $\vec{P}_i$) mathematisch wie folgt verknüpft:

$$\vec{Z} \approx \sum_{l=1}^{p} a_l \cdot \vec{P}_l \qquad\qquad (\text{Gl. 1})$$

Wie im Patentanspruch 1 weiter ausgeführt, kann man, bevor die Hauptachsenanalyse bei den Zahnvektoren durchgeführt wird, den Vektorraum so verschieben, das der Mittelwert 0 ergibt. Dies erhält man durch Differenzbildung zwischen den einzelnen Zahnvektoren und dem Durchschnittszahn. Die entstandenen Differenzvektoren können dann ebenfalls durch Hauptkomponentenverfahren analysiert werden. Insgesamt bekommt man durch diese Verfahren mit wenigen veränderbaren Parametern eine ausreichend effiziente Beschreibung neuer Zahnformen, die sich als Linearkombinationen dieser neuen Parameter (Linearfaktoren) und Hauptachsen darstellen lassen. Der entscheidende Vorteil ist, dass bei Veränderung der Parameter mit hoher Wahrscheinlichkeit einer der vorhandenen natürlichen Zahndaten angenähert wird. Die anzufertigende Restauration wird also zahnähnlich sein und die Gefahr unschöner Kauflächen ist gebannt.

Im folgenden wird die Hauptachsenanalyse bei den Zahnvektoren für den Fall genauer beschrieben, für den der Durchschnittszahn subtrahiert wird, d.h. der Vektorraum der Zähne so verschoben wird, dass der Mittelwert 0 ergibt. Damit sind auch nach der Hauptachsenanalyse die Mittelwerte der Hauptachsen (Eigenvektoren) 0. Es wird von jedem Zahnvektor $\vec{D}_j$ der Durchschnittszahn $\vec{D}$ abgezogen und eine neuer Differenzvektor $\vec{\Delta}_j$ gebildet:

$$\vec{\Delta}_j = \vec{D}_j - \vec{D}$$

Die Hauptachsenanalyse liefert dann die Eigenwerte $\lambda_k$ mit den zugehörigen Hauptachsen (Hauptkomponenten, Eigenvektoren) $\vec{P}_k$, k=1,...m. Folgende Eigenschaften ergeben sich:

1. Die Eigenwerte $\lambda_k$ entsprechen den Varianzen in Richtung der Hauptachse $\vec{P}_k$

2. Die Summe der Eigenwerte $\lambda_k$ entspricht der Summe de Varianzen von $\vec{\Delta}_j$, also der gesamten Varianz von $\vec{\Delta}_j$. Nachdem eine Mittelwertverschiebung keinen Einfluß auf die Varianz der Werte hat, entspricht daher die Summe der Eigenwerte $\lambda_k$ der Gesamtvarianz von $\vec{D}_j$.

3. Der Anteil einer Hauptkomponente $\vec{P}_k$ an der Gesamtvarianz der Datensätze ist gegeben durch:

$$\lambda_k \Big/ \sum_{l=1}^{m} \lambda_l$$

4. Der Anteil der ersten p Hauptkomponenten $\vec{P}_k$ an der Gesamtvarianz ist analog gegeben durch:

$$\sum_{l=1}^{p} \lambda_l \Big/ \sum_{l=1}^{m} \lambda_l$$

Bei den Oberkiefermolaren zeigt sich zum Beispiel, daß die ersten 7 Hauptkomponenten ca. 70% der Gesamtvarianz von 170 Zähnen beschreiben.

Ein Großteil aller möglichen Zahnoberflächen $\vec{Z}$ läßt sich nun relativ genau annähern durch eine Linearkombination der ersten p Hauptkomponenten $\vec{P}_k$ ($\alpha_i$ sind die Linearfaktoren):

$$\vec{Z} \approx \vec{D} + \sum_{l=1}^{p} \alpha_l \cdot \vec{P}_l \qquad\qquad (\text{Gl. 2})$$

Wenn man sinnvolle Randbedingungen an die Parameter $\alpha_i$ (Gl. 1) bzw. $\alpha_i$ (Gl. 2) stellt (z.B. dass der neue Zahn sich innerhalb des von den vorhandenen Zähnen aufgespannten Raumes befindet oder zumindest nicht allzu weit entfernt liegen sollte), wird jede beliebige Linearkombination nach (Gl. 1) oder (Gl. 2) wiederum einen Zahn beschreiben. Ein Zahndatensatz, der allgemein durch eine Linearkombination von Hauptachsen und evtl. Addition von Durchschnittszahn erzeugt wird, wird in dieser Patentschrift als generischer Zahnmodelldatensatz oder als generisches Zahnmodell bezüglich des betrachteten Zahntyps bezeichnet. Synonym dazu und im abstrakten Sinne wird ebenfalls in dieser Patentschrift der generische Zahnmodelldatensatz oder das generische Zahnmodell bezüglich des betrachteten Zahntyps aufgefasst als eine Kombination von Datensätzen der ausgewählten Hauptachsen und evtl. dem Durchschnittszahn. Diese Kombination kann man sich physisch z.B. vorstellen entweder als einzelne Datensätze, die durch Verknüpfungen oder Verweise verbunden sind, oder durch Zusammenfügung zu einem großen Datensatz. Wird nun eine Repräsentation dieses generischen Zahnmodells oder generischen Zahnmodelldatensatzes gewünscht, müssen nur die speziellen Linearfaktoren mit den Hauptachsen multipliziert und evtl. der Durchschnittszahn addiert werden. Das generische Zahnmodell oder der generische Zahnmodelldatensatz (im folgenden zum Teil auch als "generischer Zahn" abgekürzt) stellt also eine Art mathematische Beschreibung des gesamten Zahnraumes des entsprechenden Zahntyps dar.

Der Rekonstruktionsvorgang für den reparaturbedürftigen Zahn bzw. der Defektsituation lässt sich mittels des Durchschnittszahnes oder des generischen Zahnmodells durchführen und auch weitgehend automatisieren. Rekonstruktion bedeutet die vollständige oder zumindest teilweise Wiederherstellung der fehlenden Außenhülle des reparaturbedürftigen Zahnes oder der Defektsituation. Beim reparaturbedürftigen Zahn kann es sich um Inlay-, Onlay-, Overlay-, Teilkronen-, Kronen-, Brückenpräparationen etc. handeln, bei der Defektsituation geht es um das Auffüllen von Bereichen mit fehlenden Zähnen, z.B. Brückenzwischenglieder, Implantataufbauten oder Teilen von Teilprothesen bzw. Totalprothesen. Bei dem Begriff der Restgebisssituation handelt es sich in dieser Patentschrift um die vermessene Information (insbesondere Datensätze) von präpariertem Zahn oder Zähne (reparaturbedürftiger Zahn oder Zähne) oder Defektsituation und die zusätzliche wahlweise Einbeziehung von vermessener Information der Restzahnsubstanz, des Gegenkiefer, des funktionellen und statischen/okklusalen Bissregistrats, des Nachbarzahns/ der Nachbarzähne und/oder des Zahnfleischanteils bzw. des Kieferkamms. Beim Gegenkiefer ist in der Regel nur die Einbeziehung von einem oder mehreren Gegenzähnen, d.h. dem Zahn oder der Zähne, die dem reparaturbedürftigen Zahn oder der Defektsituation gegenüberliegen, ausreichend. Der Begriff Gegenzahn ist synonym zum Fachbegriff Antagonist zu stehen. In dieser Patentschrift werden jedoch zusätzlich unter dem Begriff Gegenzahn auch Teile des Gegenkiefers oder der gesamte Gegenkiefer subsumiert. Wählt man an der betreffenden Präparation bzw. Defektsituation und umgebender Restgebisssituation bestimmte Konstruktionspunkte bzw. Korrespondenzpunkte oder Korrespondenzstrukturen, z.B. Höckerspitzen oder Randleistenpunkte auf Restzahnsubstanz und/oder mögliche Kontaktpunkte mit dem Gegenzahn oder Nachbarzahn (Fig. 9 - 11), so kann bei Kenntnis der entsprechenden Korrespondenzpunkte und -Strukturen am generischen Zahnmodell, Durchschnittszahn etc. die Rekonstruktion bestmöglich durch Optimierungsprozesse durchgeführt werden. Beim Durchschnittszahn werden in der Regel Rotations-Translations, Skalierungs- und evtl. auch affine Transformationsparameter durch Minimierungsprozesse ermittelt. Beim generischen Zahn erfolgt zusätzlich noch die optimierte Anpassung der Parameter (Linearfaktoren) der Hauptachsen so, dass das Einfügen des generischen Zahnes, der entsprechend der Parameter verändert wurde, bestmöglich erfolgt. Wahlweise können in diesen Prozeß auch Nebenbedingungen eingebaut werden, wie z.B. Begrenzung der Größe der Parameter, damit das Ergebnis nicht weit außerhalb des Zahnraumes liegt, oder die Bedingung, dass die Gegenkaufläche oder funktionelles Registrat nicht durchdrungen werden darf, an den Kontaktpunkten sich aber berührt. Auch können Qualitätsparameter wie minimale Schichtstärken für ein Material oder belastungsoptimiertes Oberflächendesign mit berücksichtigt werden.

Neben den einzelnen Korrespondenzpunkten können aber auch alle vorhandenen Restzahnflächen (z.B. bei Inlays, Onlays, Teilkronen), oder auch Korrespondenzstrukturen, d.h. bestimmte charakteristische Areale und Formen, in ihrer Gesamtheit hergenommen und alle Punkte dieser Restzahnflächen und/oder Strukturen zur Korrespondenz genommen werden. Dies kann z.B. analog zu oben wiederum mit dem Verfahren des optischen Flusses durchgeführt werden. Ein andere Möglichkeit ist das Matching mit Optimierung der Parameter entsprechend einer Gütefunktion (z.B. Abstandsfunktion). Entscheidend dabei ist wiederum, das der Zahn nicht irgendwie deformiert wird, sondern entlang den Hauptachsen und damit im Bereich der Form natürlicher Zähne bleibt.

Im allgemeinen werden die generische Kaufläche und Datensätze der Defektsituation bzw. des reparaturbedürftigen Zahns nicht im gleichen Koordinatensystem liegen. Daher müssen bei der generischen Kaufläche neben den Parametern entlang der Hauptachsen (Linearfaktoren) zumindest auch die Rotation und Translation bestimmt werden. Die Einbeziehung einer Skalierung ist möglich, aber hier nicht unbedingt sinnvoll, da dieser Faktor bereits in der Hauptachsenrepräsentation integriert sein sollte. Eine Möglichkeit der Problemlösung besteht darin, den Anpassungsprozess in zwei Stufen zu durchlaufen:

1. Rotation und Translation des Durchschnittszahnes in das Koordinatensystem des Defektzahnes anhand der Korrespondenzpunkte und/oder Restzahnsubstanz. Dies kann mit z.B. dem Algoritmus nach Umeyama (Umeyama S.: Least-squares estimation of transformation parameters between two point patterns. IEEE PAMI 13(4); 276-280, 1991) durchgeführt werden, wobei man den Skalierungsfaktor gleich 1setzt.

2. Verbesserung der Anpassung der Korrespondenzpunkte durch Optimierung der Hauptachsenparameter (evtl. ergänzt durch lineare Faktoren der Rotation und Translation etc.).

Der Vorteil ist, dass man für beider Stufen direkte Lösungsverfahren einsetzen kann. Im allgemeinen Fall (auch einstufige Lösung) können natürlich auch bekannte nichtlineare iterative Lösungsverfahren eingesetzt werden (z.B. Gradientenabstiegsmethode, Levenberg-Marquardt etc.).

Wurde der ursprüngliche Datensatz der Restzahnsubstanz und/oder Korrespondenzpunkten in das Koordinatensystem des Durchschnittszahnes translatiert und rotiert, bestehen mit den Eigenschaften der generischen Zahnoberfläche bestmögliche Ausgangsbedingungen für die Rekonstruktion von Zahnoberflächen. Die Aufgabe besteht darin, die Parameter (Linearfaktoren) $\alpha_l$ so zu bestimmen, daß die sich ergebende Linearkombination (d.h. neue Kaufläche) möglichst gut an die vorhandene Situation anpasst. Dies erfolgt z.B. durch Minimierung einer Fehlerfunktion.

Eine weitere Optimierung der Anpassung besteht darin, nur solche Linearkombinationen zuzulassen, die eine sehr hohe Wahrscheinlichkeit aufweisen, d.h. die möglichst typische Zahnformen des Zahnraums bevorzugen. Damit soll das Ergebnis mit hoher Wahrscheinlichkeit in der konvexen Hülle der Zahndaten liegen. Es ist in diesem Zusammenhang alternativ denkbar, eine wahrscheinlichkeitstheoretische Betrachtungen mit einzubeziehen. Folgende Bedingungen sollten berücksichtigt werden:

a) Die gesuchte Kaufläche sollte im Raum der Zahnoberflächen eine möglichst hohe Wahrscheinlichkeit aufweisen, d.h. ihre Form sollte möglichst typisch für eine Kaufläche sein.

b) Die gemessenen Punkte können Meßfehler aufweisen (z.B. durch Messung oder durch Anklicken). Damit ein Meß- oder Bearbeitungsfehler bei der Auswahl der Kaufläche nicht übermäßig gewichtet wird, wird man auch hier eine Wahrscheinlichkeit für einen Messpunkt, abhängig vom Rauschen oder Fehlerquellen, berücksichtigen.

Ein solcher Ansatz könnte zu folgender Maximierung der Wahrscheinlichkeit führen:

$$P(\vec{c} \mid \vec{z}_{real}) = const \cdot P(\vec{z}_{real} \mid \vec{c}) \cdot P(\vec{c})$$
$$= const \cdot e^{-\frac{1}{2\sigma^2}\left\|\mathbf{M}\vec{c} - \vec{z}_{real}\right\|^2} \cdot e^{-\frac{1}{2}\left\|\vec{c}\right\|^2}$$

Diese Wahrscheinlichkeit wird maximal, wenn die Gütefunktion E minimal wird:

$$E = \left\|\mathbf{M}\vec{c} - \vec{z}_{real}\right\|^2 + \gamma \cdot \left\|\vec{c}\right\|^2 = \min, \qquad \gamma = \frac{1}{\sigma^2}$$

mit

$$\vec{z} = \sum_{l=1}^{p} \lambda_l c_l \vec{p}_l = \mathbf{M}\vec{c} \ ,$$

mit der Matrix $\mathbf{M} = (\lambda_1\vec{p}_1, \lambda_2\vec{p}_2, ..., \lambda_p\vec{p}_p)$, und der Messfehler mit einer Varianz von $\sigma^2$.

Die ermittelte optimale generische Zahnoberfläche wird sich schon sehr gut in die gegebene Restgebisssituation einfügen. Bei der Restgebisssituation handelt es sich um die vermessene Information (insbesondere Datensätze) von präparierten Zahn inkl. Restzahnsubstanz, Gegenkiefer, funktionelles und statisches Bissregistrat, Nachbarzähne und/oder auch vom Zahnfleischverlauf und Kieferkamm. Allerdings werden sich in der Regel noch kleinere Differenzen ergeben, wie z.B. kleine Stufen oder Lücken beim Übergang zur Restzahnsubstanz, zu hohe Stellen, die das Bissregistrat oder den Nachbarzahn durchdringen, noch fehlende Kontaktpunkte etc. Ausserdem müssen unter Umständen noch fehlende Flächenanteile wie Approximalflächen, Oral- und Vestibulärflächen ergänzt werden. Diese zusammengefasst als Anpassung bezeichneten Vorgänge, die meistens nur geringfügige Änderungen beinhalten, liefern dann den fertigen Datensatz, der für die Steuerung einer Maschine benutzt wird.

In Patentanspruch 4 wird die Verwendung dieser ermittelten Datensätze für die physische Herstellung beschrieben. Im Prinzip sind alle möglichen automatisierten Fertigungsverfahren wie CNC-Fräsen oder -schleifen, Laserbearbeitung, Stereolithographie oder lithographische Sinterverfahren einsetzbar. Die Materialpalette für die Zahnrestauration, Zahnersatzteile oder Zahnmodelle kann von Kunststoff über Metalle (Titan, Gold, Stahl etc.) bis zu Keramik reichen. In der Zahnmedizin sind bereits eine Reihe von Materialien speziell für den CAD/CAM-Prozess verfügbar.

Patentanspruch 5 beinhaltet den gesamten Herstellungsprozess von Vermessung bis Produktion.

Ausführungsvarianten und Erklärungen der Patentansprüche 6 bis 9 sind bereits vorher beschrieben worden. Patentanspruch 10 bezieht sich explizit auf die Berücksichtigung von funktionellen und/oder statischen bzw. okklusalen Bissregistraten. Ein großer Vorteil der gesamten Kauflächenanpassung mittels mathematischer bzw. elektronischer Vorgehensweise besteht darin, dass man die gesamte Herstellungskette von Gegenkieferabformung, Herstellung eines Gipsmodells dieses Gegenkiefers, Einartikulation des Gegenkiefers und Zuordnung zum gesägten oder Präparationsmodell, bis zur Bestimmung und Justierung der Kiefergelenksparameter etc. nicht mehr durchführen muss. Die Alternative stellt hier die direkte Abformung der Gegenkiefersituation mittels Bissregistrate im Mund dar. Das statische Bissregistrat, manchmal auch als okklusales Bissregistrat bezeichnet, erhält man durch Einbringung von Abformmaterial an die gewünschte Stelle, indem der Patient zubeißt und die Zähne zugebissen lässt, bis das Material abbindet. Über die Kieferbewegungen erhält man Auskunft, indem vor dem Abbindens des eingebrachten Abdruckmaterials der Patient zusätzlich noch möglichst viele verschiedene Kieferbewegungen ausführt. Dies ergibt dann das funktionelle Bissregistrat, manchmal auch als FGP (function generated path) bezeichnet. Mit dieser Vorgehensweise erhält man sehr genaue 3D-Informationen über die Bahnen des der Präparation gegenüberliegenden Zähnen, und damit auch Begrenzungslinien und Konstruktionshinweise, wo Kontaktpunkte liegen könnten und wo die rekonstruierte Zahnoberfläche nicht ausgedehnt werden darf, bzw. welches die höchsten Stellen sein können. In Patentanspruch 11 wird genau diese Information für die Korrespondenzfindung und damit für eine genauere Adaptation des generischen Zahnes herangezogen. Durch geeignete mathematische Formulierung kann diese Information in Form von Randbedingungen in die Optimierungsbzw. Minimierungsverfahren einbezogen werden. Diese Bedingung könnte z.B. lauten: Kontaktpunkte sind Berührungspunkte (Interpolation des Punktes mit zweiter Ableitung gleich 0) mit dem Bissregistrat, während die restlichen Bereiche von der rekonstruierten Fläche nicht berührt werden dürfen.

In Patentanspruch 12 wird eine Möglichkeit der Automatisierung der Kontaktpunktfindung mit dem Gegenzahn (Antagonisten) beschrieben. Durch Vergleich des statischen (okklusalen) Bissregistrats mit dem funktionellen Bissregistrat, die beide für die entsprechende Situation wie oben ausgeführt vom Patienten genommen wurden und sich als vermessene Datensätze im gleichen Koordinatensystem befinden (referenziert sind), sind die Bereiche, bei denen das eine Bissregistrat einen geringen Abstand von dem anderen Bissregistrat hat oder sich beide berühren, besonders ausgezeichnet. Diese Areale stellen die möglichen Kandidaten für die Kontakte mit den Antagonisten dar, in den anderen Bereichen können keine Kontaktpunkte liegen. Wenn man weis, wo sich die korrespondierenden Kontaktpunkte auf der generischen Zahnoberfläche befinden, kann man die Optimierung der Linearfaktoren weitestgehend automatisieren.

In Patentanspruch 13 werden zusätzlich für die Approximalflächengestaltung (z.B. Lage des Approximalkontaktes, Ausdehnung etc.) und für die Auswahl von Korrespondenzpunkten bzw. strukturen (z.B. Randleisten, Formen der Kaufläche etc.) die vermessenen Informationen der Nachbarzähne einbezogen. Ebenso können einzelne Punkte (z.B. Kontaktpunkte) oder die Form und Strukturen des Gegenzahnes für die Korrespondenzbildung ausgenutzt und damit die Auswahl der bestpassendsten Zahnoberfläche für die Rekonstruktion des reparaturbedürftigen Zahnes bzw. Defektsituation durchgeführt werden. Ebenso könnte die Information des entsprechenden symmetrisch gegenüberliegenden Zahnes herangezogen werden, da man oft davon ausgeht, dass diese Zahnformen nur spiegelbildlich sind, sich aber sonst sehr stark ähneln. Insbesondere beinhaltet dieser Anspruch die Möglichkeit, die aus der Hauptachsenanalyse bzw. Korrespondenzanalyse gefundenen Zusammenhänge zwischen Nachbarzähnen des gleichen Patienten (z.B. bei der Herstellung des generischen Zahnmodells von benachbarten Zähnen) aus der Information des Nachbarzahnes/ der Nachbarzähne auf die zu ergänzende Außenhülle oder zumindest Teile dieser Außenhülle zu schließen. Eine Möglichkeit besteht in der Optimierung der Parameter des kombinierten generischen Zahnmodelldatensatzes bei der Anpassung an den Nachbarzahn/Nachbarzähne, wobei gleichzeitig die zu rekonstruierende Zahnoberfläche entsprechend verändert wird. Das gleiche Verfahren ist auch für den Gegenzahn bzw. den symmetrisch gegenüberliegenden Zahn anwendbar. Insbesondere wird in diesem Anspruch auch darauf hingewiesen, dass die Information von Nachbarzahn/Nachbarzähnen, von Gegenzahn und/oder vom symmetrisch gegenüberliegenden Zahn/Zähnen auch aus zweidimensional vermessenen Datensätzen bestehen kann. Ausgehend von diesen Datensätzen kann man unter Zuhilfenahme eines entsprechenden generischen Zahnmodells durch Optimierung von Abbildungs-, Beleuchtungs-, Render- und/oder Projektionsfunktionen auf die dreidimensionale Struktur schließen (siehe z.B. Blanz, V., Romdhani, S.: Face Identification across different poses and illuminations with a 3D morphable model. Proc. Int. Conference on Automatic Face and Gesture Recognition, 202-207, 2002) und diese wiederum für die Rekonstruktion ausnutzen. Der Vorteil dieser zweidimensionalen Vermessung liegt darin, dass man relativ einfach z.B. mittels einer intraoralen Kamera oder eines Fotoapparats am Patienten diese Aufnahme bzw. Datensätze erstellen kann.

Noch notwendige Anpassungen können durchgeführt werden, sofern noch Störstellen und Interferenzen nach Berechnung des bestpassendsten generischen Zahnes oder Durchschnittszahnes vorhanden sind. Dies können kleine Stufen oder Lücken beim Übergang zur Restzahnsubstanz, zu hohe Stellen, die das Bissregistrat oder den Nachbarzahn durchdringen, noch fehlende Kontaktpunkte etc sein. Hier bieten sich Verfahren an, die gewährleisten, dass die Veränderungen lokal begrenzt und möglichst klein bleiben und gleichzeitig einen harmonischen und glatten Übergang zu den nicht veränderten Bereichen ergeben. Dies kann durch bekannte Deformations und/oder Morphing-Verfahren erfolgen. Außerdem müssen unter Umständen noch fehlende Flächenanteile wie Approximalflächen, Oral- und Vestibulärflächen ergänzt werden. Die möglichen Verfahren einer automatisierten Ergänzung dieser Flächen werden weiter unten beschrieben. Insgesamt können diese Prozesse automatisch oder interaktiv erfolgen. Bei der interaktiven Manipulation kann der Zahnarzt oder Zahntechniker nach seiner jeweiligen Vorstellung die Gestaltung noch optimieren. Diese Möglichkeit sollte in Verfahren zur Herstellung von Zahnersatzteilen oder Zahnrestaurationen in der Regel immer implementiert sein.

Mit Hilfe des generischen Zahnes gelingt die Verwirklichung verschiedener Okklusions- und Funktionskonzepte. In der Zahntechnik gibt es verschieden Theorien, wo statische und funktionelle Kontaktpunkte zum Nachbarzahn oder Antagonisten liegen sollen. Der generische Zahn bietet die Möglichkeit, quasi online zu entscheiden, welches Konzept man verwenden möchte und wo die Kontaktpunkte liegen sollten (Fig. 9-11). Dabei wird z.B. entweder einmal für einen bestimmten Benutzer oder Labor, der oder das ein bestimmtes Konzept favorisiert, oder auch vor jeder neuen Versorgung die gewünschten Kontaktpunkte auf dem generischen Zahn markiert, die entsprechenden Korrespondenzpunkte auf dem Bissregistrat/ und oder Restzahnsubstanz bzw. Nachbarzahn, wie in den Patentansprüchen 14 und 15 ausgeführt. Durch Anpassung der Parameter bezüglich der korrespondierenden Punkte erhält man nach Minimierungsverfahren wiederum eine funktionell gestaltete natürliche Kaufläche. Dieses Verfahren geht nur mit generischen Zähnen, da im Falle von Zahnbibliotheken der beste Zahn nur ausgewählt werden kann, wenn bei Veränderung der Kontakt/Funktionssituation die entsprechenden Referenzpunkte aller Zähne neu bestimmt werden müssen, bei hoher Anzahl von Zähnen ein aufwendiges Unterfangen. Auf der anderen Seite ist bei Deformation nur eines Modellzahnes, der nicht auf Basis eines generischen Zahnes erzeugt ist und die Hauptkomponentenanalyse nicht durchgeführt wurde, nicht gewährleistet, dass das Ergebnis ein harmonisches, zahnähnliches Resultat ergibt.

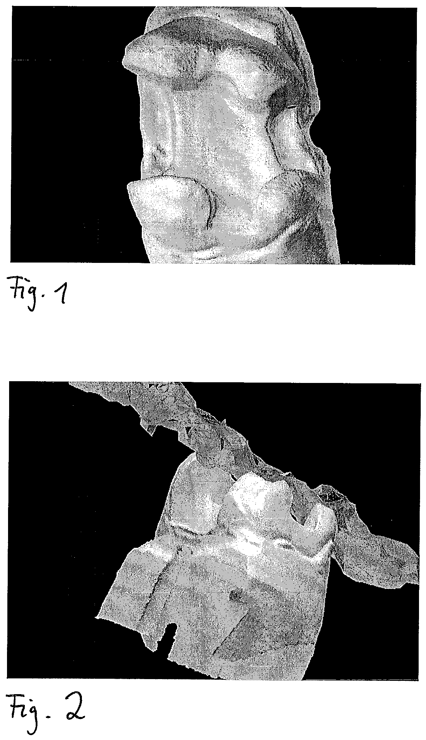

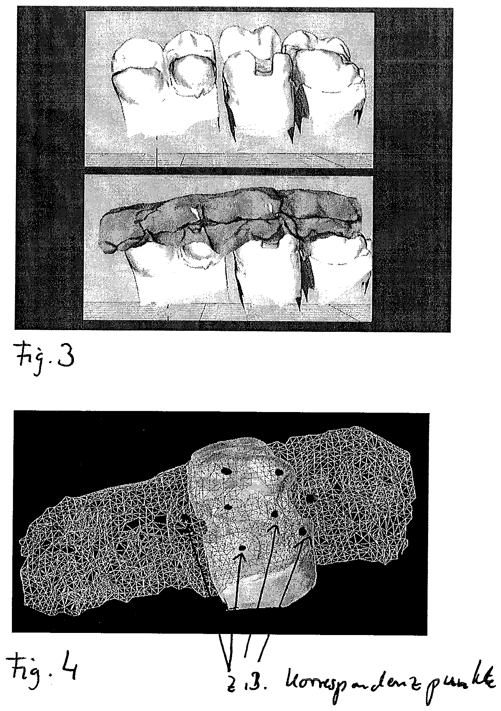

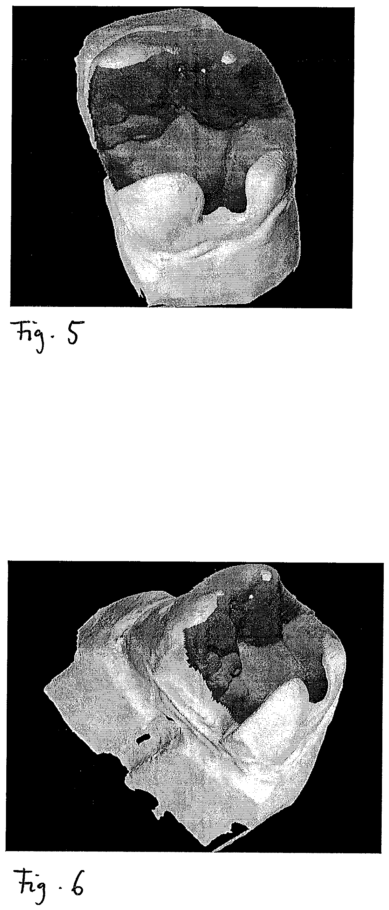

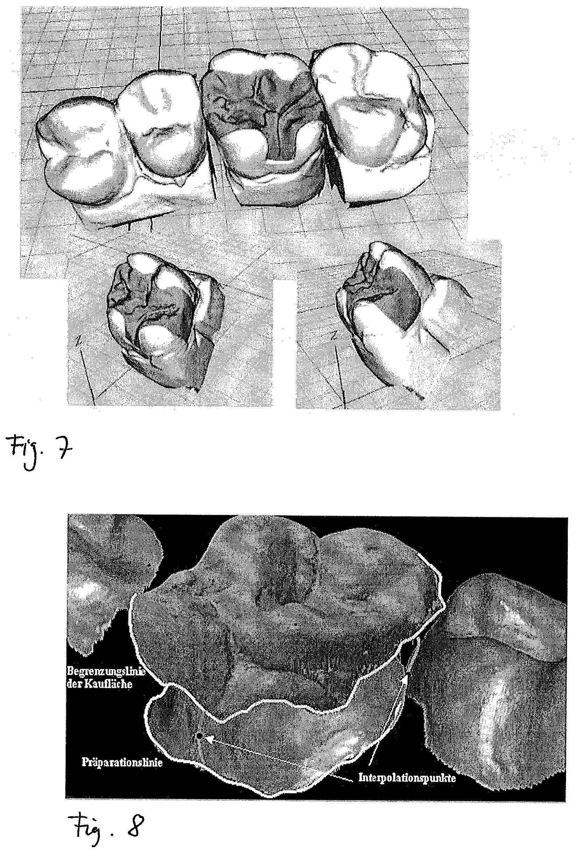

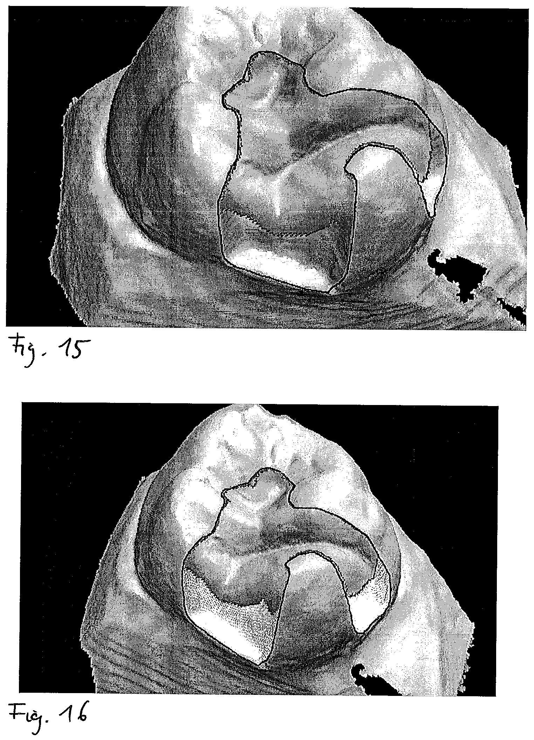

Ausgehend von 3D-Datensätzen der Gegenkiefersituation (Fig.2) und der Präparation (Fig. 1) bzw. mehreren Präparationen, die zueinander referenziert sind, werden Zahnersatzteile dadurch hergestellt, dass nach Referenzierung die vorhandenen Bissregistrate mit den Päparationsdatensätzen anhand der möglichen Überlappbereiche (Fig. 3) nach Auswahl geeigneter Korrespondenzpunkte (Fig. 4) die am besten passende Kaufläche aus einer Zahnbibliothek automatisch ausgewählt wird (Fig. 5). Ein Fehler-Minimierungs-Verfahren für die Auswahl und Anpassung der Bibliothekskaufläche, das sich hierfür sehr gut eignet und nicht iterativ erfolgt, wird z.B. bei Umeyama (Umeyama S.: Least-squares estimation of transformation parameters between two point patterns. IEEE PAMI 13(4): 276-280, 1991) beschrieben. Anschließend werden vorhandene Interferenzen bzw. Überschneidungen mit der Gegenzahnreihe und /oder Nachbarzähne beseitigt und im Falle der Inlays, Onlays und z.T. Teilkronen wird noch die Restzahnsubstanz berücksichtigt werden die fehlenden Außenflächen ergänzt (Fig. 6 und 8) und wird dann an die Präparationslinie so angepasst, dass ein nahezu glatter harmonischer Übergang erfolgt (Fig. 7). Durch Verschmelzung der Außen- und Innenflächen entlang der Präparationslinie (Randkurve) kann dann das Zahnersatzteil gefräst werden. Entscheidend zum einen ist, dass im Vergleich zu vorher erwähnten bereits bekannten Methoden durch die Auswahl vieler unterschiedlicher Zähne aus einer Zahnbibliothek nicht der Zahn der Situation angepasst wird, sondern ein für die Situation schon sehr gut passender Zahn ausgewählt wird, bei dem dann nur sehr kleine und damit automatisierbare und fehlerunanfälligere Adaptationen durchzuführen sind. Der zweite Vorteil ist die Trennung von wichtigen oder komplizierten Teilen der Zahnoberfläche von weniger wichtigen oder einfacheren Teilen. Zu ersteren gehört z.B. die Kaufläche, zu zweiten die Vestibulär, Approximal- und Oralflächen der Zähne. Durch diese Aufteilung kann man sich auf die bessere Anpassung der komplizierteren Oberflächen aus der Zahnbibliothek beschränken, während die Außenflächen automatisch ergänzt und rekonstruiert werden. Für die Aussenflächen genügt die Angabe nur weniger Konstruktionspunkte (Fig. 8 und 16). Eine Möglichkeit der Implementierung ist die Berechnung von Bezier-, Nurbs- oder B-Spline-Flächen, die stetig und glatt an die entsprechenden Teile der Präparationsgrenze und der Grenze des eingefügten Bibliotheksdatensatzes anschließen und dabei die Konstruktionspunkte (wie z.B. Approximalkontakt, Ausbuchtung der Vestibulär- oder Oralfläche) interpolieren. Die Zahnbibliothek kann mit einer Struktur aufgebaut sein, bei der jedem Zahndatensatz entweder durch Referenzierung oder durch entsprechender Namensgebung ein Datensatz zugeordnet ist, der den Typ und die Merkmale, die für die Auswahl berücksichtigt werden sollen, beinhaltet. Zusätzlich soll die Bibliothek aus Zahnoberflächen bestehen, die von natürlichen kariesfreien und unversehrten Zähnen herrühren.

Die allgemeinste Form der Zahnbibliothek enthält die Gesamtheit aller möglichen vorkommenden natürlicher und auch künstlicher Zahnformen. Sinnvollerweise wird man die Zahnbibliothek in Gruppen mit unterschiedlichen Zahntypen einteilen. Bei dieser Untergruppierung nach dem Zahntyp kann es sich zum Beispiel um Molaren, Prämolaren, Eckzähne und Frontzähne handeln. Als Zahntyp kann aber auch der OK-6er, UK-4er, OK-1er etc. stehen. Möglich ist des weiteren eine Unterscheidung nach Alter und Abrasion, nach Geschlecht, nach Volkszugehörigkeit, nach Größe der Zähne, nach morphologischen Besonderheiten etc, z.B. können die Gruppen OK-7er im Alter von 50-60 Jahren, OK-6er mit und ohne Tuberculum Carabelli, UK-3er bei weiblichen Personen ein Beispiel für einen Zahntyp darstellen. Der Begriff Zahntyp umfasst also eine je nach Aufgabenstellung sehr variable Gruppierungsmöglichkeit.

Patentanspruch 16 beschreibt ein Verfahren, in dem man bei der Erstellung des generischen Zahnmodelldatensatzes den Faktor Alter bzw. Abrasionsgrad, wobei Zahnbibliotheksflächen eines bestimmten Zahntyps in allen Alters- bzw. Abrasionsstufen vorliegen sollen, berücksichtigt und den oder die ermittelten Kombinationen aus Linearfaktoren und Hauptkomponenten, die diesen Faktor beschreiben, nutzt, um die Abrasion für die jeweilige Restgebissituation optimal einzustellen.

Patentanspruch 17 zeigt eine neue Möglichkeit der Herstellung von Zahnrestaurationen auf, bei der automatisch ein Vorschlag für die möglichen Lokalisationen aller Berührungspunkte mit dem Gegenzahn/Gegenbezahnung (d.h. den Berührungsstellen mit dem Gegenkiefer) ermittelt wird. Dazu wird ein funktionelles Bissregistrat und ein statisches bzw. okklusales Bissregistrat vermessen, die Datensätze im gleichen Koordinatensystem referenziert, so dass es der Situation am Patienten bzw. am Modell entspricht und anschließend alle Bereiche oder Punkte, die einen sehr geringen Abstand von dem einen zum anderen Registrat aufweisen, herausgefiltert. Entscheidend ist, dass außerhalb dieser Bereiche kein Kontaktpunkt liegen kann und darf. Daher könnte sogar die Kontaktpunktgestaltung automatisiert oder zumindest wesentlich vereinfacht werden.

Die Datensätze des Durchschnittszahns, des generischen Zahndatensatzes, der rekonstruierten Zahnersatzteile, der Zahnrestaurationen oder der Zahnmodelle können durch Glättung (Filterung) oder spezielle Anpassung an die Werkzeug- bzw. Bearbeitungsgeometrien für den Herstellungsprozess aufbereitet werden. Darunter fallen auch Fräserradiuskorrekturen etc.

Alle vorgestellten Verfahren eignen sich für Inlay-, Onlay-, Teilkronen-, Kronen- und Brückenversorgungen gleichermaßen. Ein weiterer Vorteil, wie in Patentanspruch 28 ausgeführt, liegt darin, dass ausgehend von der rekonstruierten Kaufläche auch eine reduzierte Kauflächengestaltung für Gerüste möglich ist, die gewährleistet, dass die Verblendung nachher immer ungefähr die gleiche Schichtstärke aufweist. Dies kann man erreichen, indem in einem konstanten Abstand von der rekonstruierten Oberfläche die neue Oberfläche berechnet wird oder zumindest durch Abflachung im Bereich der Höcker und Fissuren die Kaufläche dann entsprechend der Schichtstärke in Richtung des präparierten Zahnes verschoben wird.

Mit Hilfe einer numerisch gesteuerten Maschine, gesteuert von den ermittelten Datensätzen, erfolgt die physische Herstellung von Zahnmodellen, Zahnrestaurationen und Zahnersatzteilen. Im Prinzip sind alle möglichen automatisierten Fertigungsverfahren wie CNC-Fräsen oder -Schleifen, Laserbearbeitung, Stereolithographie oder lithographische Sinterverfahren einsetzbar. Die Materialpalette für die Zahnrestauration, Zahnersatzteile oder Zahnmodelle kann von Kunststoff über Metalle (Titan, Gold, Stahl etc.) bis zu Keramik reichen. In der Zahnmedizin sind bereits eine Reihe von Materialien speziell für den CAD/CAM-Prozess verfügbar.

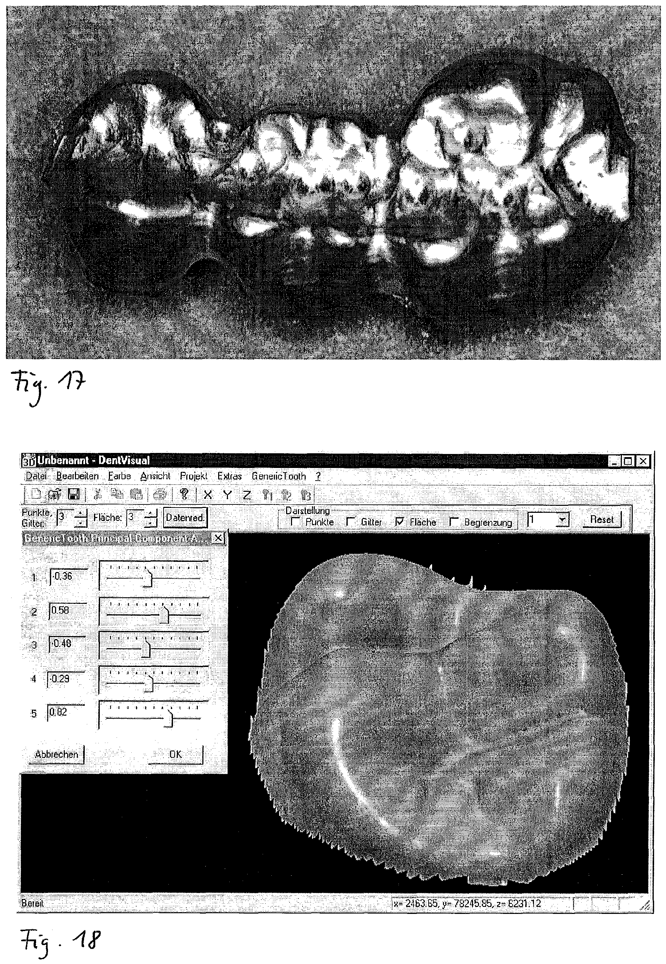

In dem Patentanspruch 18 werden Vorrichtungen beschrieben, die es ermöglichen, dass für den generischen Zahnmodelldatensatz die Linearfaktoren der zumindest wichtigsten Hauptkomponenten durch eine Regeleinrichtung direkt und interaktiv verändert werden können. Gleichzeitig kann die Auswirkung dieser Veränderung in einer bildlichen Darstellung betrachtet und analysiert werden. In Fig. 18 ist eine Form der Ausgestaltung zu sehen. Die erwähnten Vorrichtungen können z.B. eingesetzt werden, um anstelle der automatischen Rekonstruktion und Optimierung dem Zahnarzt oder Zahntechniker die Möglichkeit zu geben, interaktiv nach eigenen Vorstellungen den generischen Zahnmodelldatensatz an die Restzahnsituation anzupassen.

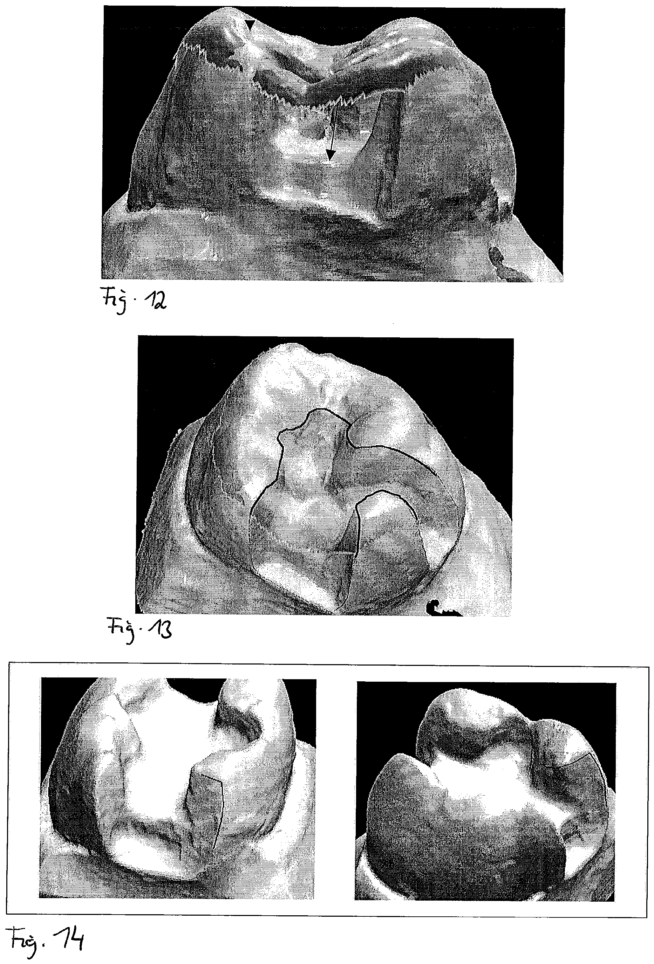

Mit Verfahren kann man die gesamten Kauflächenrekonstruktionen durchführen, ohne dabei explizit die Restzahnsubstanz ausschneiden oder besonders markieren zu müssen. Vielmehr wird der vollständige Datensatz des reparaturbedürftigen Zahnes herangezogen (Fig. 12). Durch Anklicken weniger Startwerte (Korrespondenzpunkte) auf der Restzahnsubstanz wird ein Vorschlag angeboten, anhand dessen für die weitere Iteration oder Anpassungsprozess nur solche Korrespondenzpunkte in Betracht kommen, die unterhalb eines gewissen Abstandes zwischen vorgeschlagener Zahnfläche und reparaturbedürftigen Zahn liegen (Fig. 12). Die Abstandsschwelle kann auch variiert oder adaptiv angepasst werden. Somit werden mit hoher Wahrscheinlichkeit bei der Rekonstruktion Punkte in der Kavität oder auf den beschliffenen Bereichen der Zahnoberfläche nicht mit berücksichtigt oder fallen durch die geringe Anzahl nicht ins Gewicht. Der Vorteil dieser Vorgehensweise besteht auch darin, dass, die automatische Ergänzung der Präparationslinie möglich wird. Nach erfolgter Rekonstruktion und Anpassung der Kaufläche wird nach den Bereichen gesucht, bei denen ein Übergang von kleineren Abstandswerten (Bereiche, wo sich noch Restzahnsubstanz befindet; hier weist in der Regel die rekonstruierte Kaufläche geringe Abweichungen auf) zu Bereichen mit größeren Abständen (Bereiche wo der Zahn beschliffen oder Zahnsubstanz entfernt wurde) auftritt. In diesen Übergangsbereichen muss auch die Präparationsgrenze oder zumindest Teile davon liegen (Fig. 13). Diese Vorgehensweise kann noch verbessert werden, wenn man in diesen Arealen nach den Stellen der stärksten Krümmung auf der Oberfläche des Datensatzes des reparaturbedürftigen Zahnes sucht und diese Stellen stärkster Krümmung in diesen Bereichen zu einer Linie verbindet (z.B. Fig. 14 und 15). Damit ist von der Rekonstruktion bis zur Präparationsgrenzenfindung ein vollautomatischer Prozess vorstellbar. Man kann dies aber auch als Unterstützung und Vorschlagsunterbreitung für eine weitere interaktive Nacharbeitung seitens des Benutzers vorteilhaft einsetzen.

Eine interaktive Möglichkeit der Eingabe der Präparationsgrenze ist möglich. Hierbei werden in bestimmten Abständen Punkte auf der Oberfläche des elektronischen Bildes des reparaturbedürftigen Zahnes angeklickt. Dieses Anklicken kann mit verschiedenen Steuer- und Kontrollelementen erfolgen, z.B. Computermaus, Tastatur, Joystick oder 3D-Maus. Zwischen diesen ausgewählten Punkten wird jeweils eine Verbindungslinie im Raum interpoliert. Damit man Punkte von der vermessenen Zahnoberfläche bekommt, wird die Verbindungslinie auf die Oberfläche projiziert (Fig. 14). Dabei ist entscheidend, dass die Projektionsrichtung für bestimmte Abschnittsbereiche oder auch für jeden Abschnitt unterschiedlich gewählt werden kann. Dies kann z.B. durch vorprogrammierte Werte erreicht werden oder durch interaktive Einstellung der Ansicht des Zahndatensatzes (Fig. 14). Vorteilhaft kann dies z.B. dadurch erfolgen, dass in der Ansicht zur Markierung bzw. Anklicken des jeweiligen Punktes auch die Projektion der Linie in der gleichen Richtung durchgeführt wird. Um einen möglichst glatten Kurvenverlauf zu bekommen, können die Verbindungslinien neben Geraden auch Spline- oder Parabelsegmente sein. Auch nach erfolgter Projektion auf die Oberfläche können Splinesegmente oder Ähnliches die eventuell zackigen oder verrauschten Kurven noch glätten. Eine besonders sinnvolle Variante besteht noch darin, in der Nähe oder auch zwischen den angeklickten Punkten nach Stellen mit den größten Krümmungen zu suchen. Aufgrund der Vorgehensweise beim Präparieren und Beschleifen eines Zahnes zur Versorgung mit einer Zahnrestauration sind dies die Stellen, wo sich die Präparationsgrenze befinden sollte. Durch Verbinden der Stellen mit den größten Krümmungen, also eine Linie der größten Krümmungen, erhält man schon einen sehr guten Vorschlag für den Verlauf der Präparationsgrenze (Fig. 15).

Es gibt Verfahren, die es ermöglichen, eventuell im Datensatz der Zahnrestauration oder Zahnersatzteilen vorkommende Fehlstellen zu finden und zu schließen. Solche Fehlstellen können z.B. dadurch entstehen, dass die rekonstruierte Kaufläche oder der rekonstruierte Datensatz nicht den gesamten beschliffenen Anteil überdeckt oder die Anpassung im Bereich der Präparationsgrenze nicht fehlerfrei erfolgt und daher der Datensatz in diesem Bereich absteht oder Fehler aufweist (Fig. 6, 8, 15). Durch automatischen Vergleich der Präparationslinie mit der Berandungskurve des rekonstruierten Datensatzes kann durch Abstandsprüfung entschieden werden, welche Bereiche der Linien bzw. Kurven zu weit auseinander liegen und daher eine Auffüllung oder Ergänzung notwendig machen (Fig. 15). Nachdem der Startpunkt für Präparationslinie und Berandungskurve nicht identisch sein müssen, müssen die in Frage kommenden Abschnitte der Berandungskurve den entsprechenden Abschnitten der Präparationsgrenze noch automatisch zugeordnet werden. Für die Berechnung der Ergänzungsfläche kann es noch notwendig werden, im Übergangsbereich von dem einen Kurvensegment zu dem anderen Kurvensegment weitere Punkte der jeweiligen Kurven hinzuzufügen, die vorher bei der Abstandsprüfung nicht dem zu ergänzenden Bereich zugeordnet werden konnten und nun ermöglichen, dass eine möglichst geschlossene Linie für die Berechnung der Ergänzungsfläche entsteht. In Patentanspruch 42 wird dann explizit ein Verfahren beschrieben, wie man diese Fehlstellen schließen kann (siehe auch Fig. 16).

Die Kontrolle des Ergebnis oder noch notwendige Interaktionen, die man dem Zahntechniker oder Zahnarzt immer ermöglichen sollte, kann für den Bediener durch Visualisierung mit 3D-Brillen oder 3D-Monitoren etc. erfolgen. Dies ist für den ungeübten Bediener vertrauter.

Die Einbeziehung der Nachbarzähne oder Antagonisten oder der symmetrisch gegenüberliegenden Zahntypen bei der Auswahl der besten Kaufläche ist mittels der generischen Kauflächen und der zugehörigen Hauptkomponenten ebenfalls möglich.

Die Erfindung ist anhand der Ausführungsbeispiele in der Beschreibung und in den Abbildungen lediglich exemplarisch dargestellt.

In den Zeichnungen zeigen:

Fig. 1 einen reparaturbedürftigen Zahn;

Fig. 2 ein zu dem reparaturbedürftigen Zahn referenziertes Bissregistrat;

Fig. 3 den Zahn gemäß Fig. 1 dargestellt mit Nachbarzähnen (oben) und zusätzlich mit refernziertem Bissregistrat (unten);

Fig. 4 eine Darstellung des Zahns nach Fig. 1 mit Bissregistrat und ausgewählten Korrespondenzpunkten;

Fig. 5 eine aus einer Zahnbibliothek anhand der Korrespondenzpunkte ausgesuchte Zahnoberfläche;

Fig. 6 rotierte Darstellung der Situation nach Fig. 5 mit erkennbaren Fehlstellen;

Fig. 7 eingepasste und vollständig ergänzte Zahnrestauration;

Fig. 8 eingepasste Zahnfläche für Kronenpräparation mit Einzeichnung von Interpolationspunkten für die Rekonstruktion der noch fehlenden Aussenflächen;

Fig. 9 ein Beispiel einer generisch erzeugten Zahnoberfläche mit Korrespondenzpunkten;

Fig. 10 reparaturbedürftiger Zahn mit den der Fig. 9 entsprechenden Korrespondenzpunkten;

Fig. 11 reparaturbedürftiger Zahn mit Bissregistrat und mit den der Fig. 9 entsprechenden Korrespondenzpunkten;

Fig. 12 Beispiel für die Unterscheidung der Bereiche, die zur beschliffenen Zahnsubstanz gehören und durch eine Zahnrestauration aufgefüllt werden müssen, und Bereichen, die zur unversehrten Restzahnsubstanz gehören, anhand der Prüfung des Abstandes während des Rekonstruktions- und Anpassungsprozesses der Ausssenhülle;

Fig. 13 Beispiel für die Erkennung der Präparationsgrenze am Übergang zwischen den beiden vorher unterschiedenen Bereichen;

Fig. 14 Beispiel für das interaktive Einzeichnen der Präparationsgrenzen unter verschiedenen Ansichten und Projektion der Verbindungslinie auf die Zahnoberfläche;

Fig. 15 Beispiel für das Auffinden noch zu ergänzender Bereiche durch Vergleich der beiden Berandungskurven;

Fig. 16 eine vollständige Zahnrestauration, bei der die noch fehlenden Areale automatisch ergänzt wurden;

Fig. 17 Beispiel für eine nach dem Verfahren des generischen Zahnmodells in einer Maschine angefertigten Zahnrestauration;

Fig. 18 ein Beispiel für eine Regeleinrichtung zur Veränderung der Linearfaktoren und der gleichzeitigen Darstellung der Veränderung;

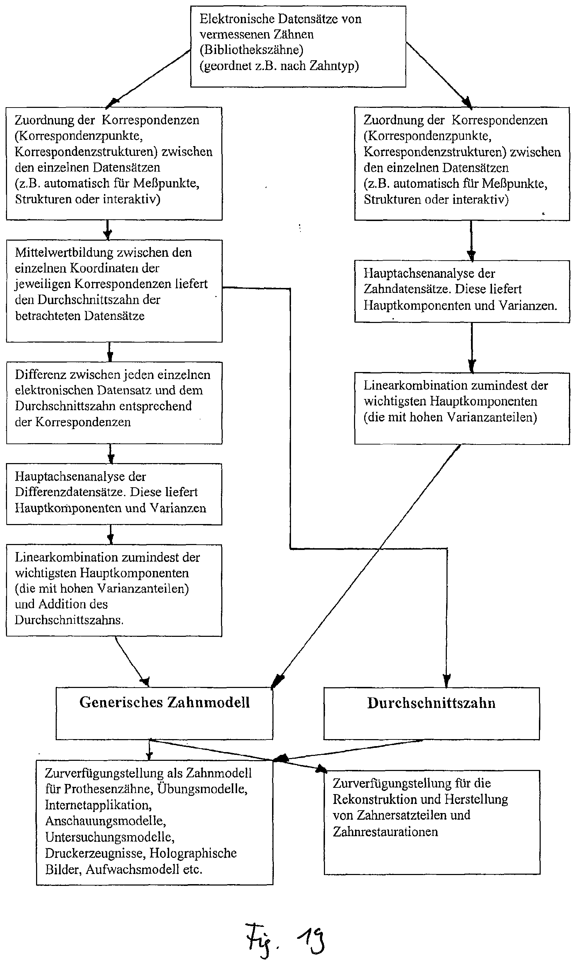

Fig. 19 ein Flussdiagramm für die Erstellung eines Durchschnittsdatensatzes oder eines generischen Zahnmodelldatensatzes;

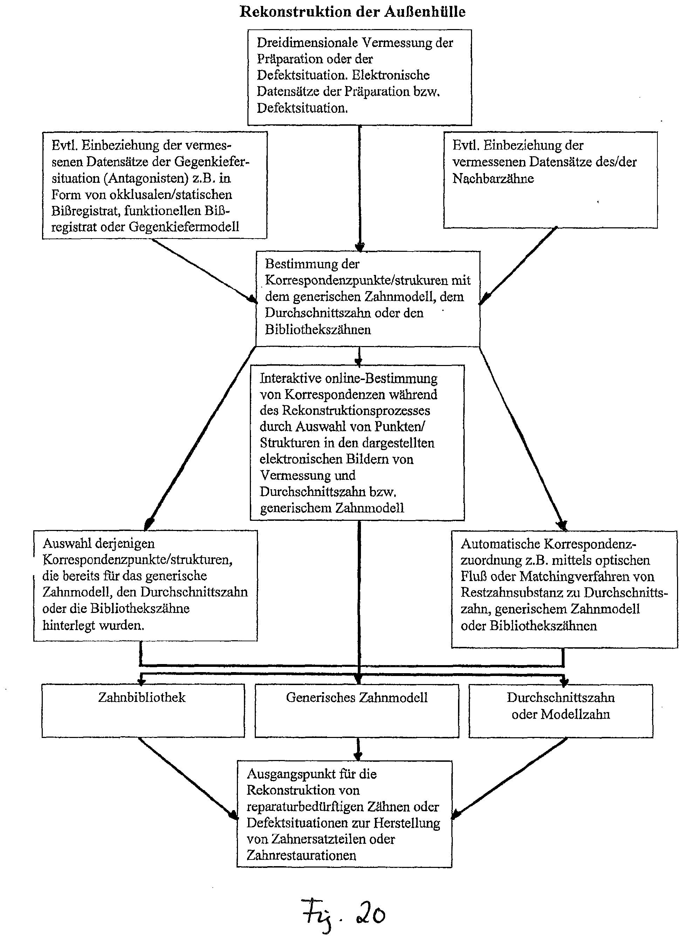

Fig. 20    ein Flussdiagramm für eine Rekonstruktion einer Außenhülle;

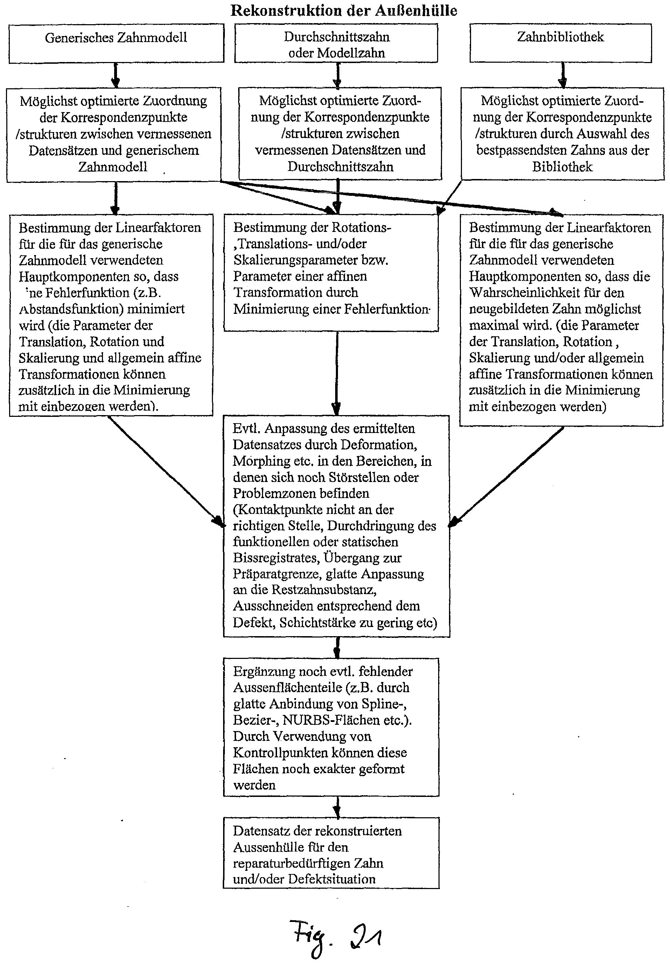

Fig. 21    eine Fortsetzung des Flussdiagramms von Fig. 14 für eine Rekonstruktion einer Außenhülle;

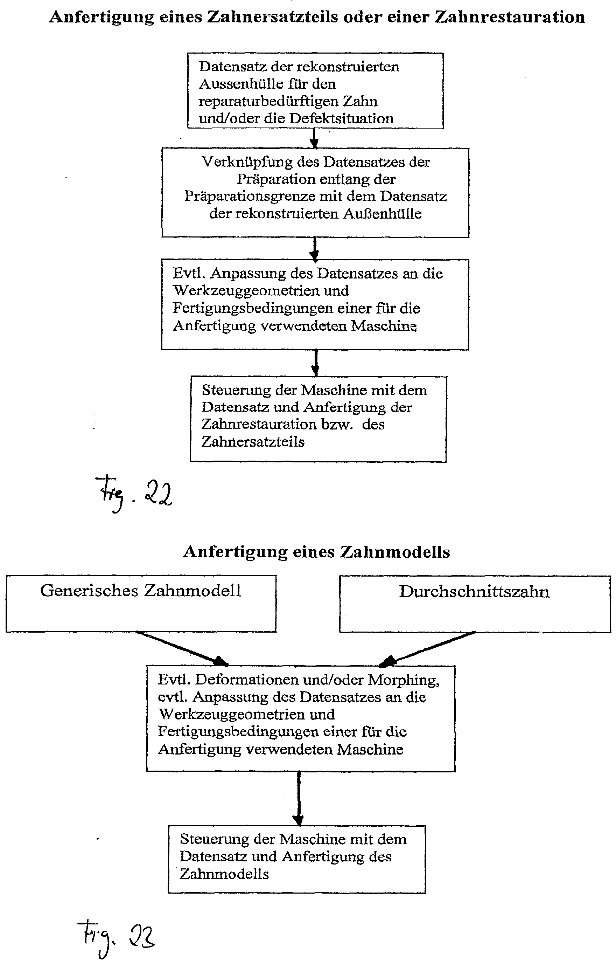

Fig. 22    ein Flussdiagramm für eine Anfertigung eines Zahnersatzteils oder einer Zahnrestauration; und

Fig. 23    ein Flussdiagramm für eine Anfertigung eines Zahnmodells.

Es folgen nun weitere Erläuterungen zur Erfindung und zu Ausführungsformen der Erfindung.

Fig. 1: Zeigt einen dreidimensional vermessenen reparaturbedürftigen Zahn als Höhendatensatz.

Fig. 2: zeigt ein zu einem reparaturbedürftigen Zahn referenziertes Bissregistrat. Dieses Bissregistrat enthält Informationen zum Antagonisten. Es kann sich dabei entweder um ein statisches Bissregistrat und/oder um ein funktionelles Bissregistrat und/oder um die Gegenzahnreihe handeln. Wichtig ist nur, dass diese Informationen ins gleiche Koordinatensystem wie der Zahn referenziert werden.

Fig.3: zeigt die gleiche Situation wie in Fig. 2, jedoch mit Nachbarzähnen (oben) und zusätzlichem Bissregistrat (unten) dargestellt. Die gesamte Anordnung stellt die Restgebisssituation dar. Die Nachbarzähne geben z.B. die Information für die mesial-distale Ausdehnung der rekonstruierten Außenhülle. Außerdem kann anhand der Form der Nachbarzähne eine Auswahl für die Zahnoberfläche (Außenhülle) getroffen werden, die für die Rekonstruktion in der entsprechenden Situation in Frage kommen.

Gemäß Fig. 4: können durch Markieren von Punkten auf der Restzahnfläche und/oder Kontaktpunkten auf dem Bissregistrat (Gegenzahnreihe) und/oder zum Approximalkontakt des Nachbarzahnes die Zahnoberflächen entweder aus der Bibliothek oder mittels des generischen Zahnes mit Hauptkomponenten optimal durch entsprechende Minimierung einer Fehlerfunktion angepaßt werden. Anstatt der Punktmarkierungen kann man auch größere Bereiche auswählen, wie z.B. Restzahnsubstanz und/oder Kontaktflächen, anhand derer die Zahnoberflächen durch Matching oder optischen Fluß angepaßt werden. In einer weiteren Ausgestaltung der Erfindung können die Lokalisationen möglicher Kontaktpunkte auch automatisch durch Vergleich des funktionellen Bissregistrates und des statischen (okklusalen) Bissregistrates ermittelt werden.

Fig. 5. zeigt eine Kaufläche aus der Bibliothek ausgesucht und an die Position transformiert oder eine generische Kaufläche, durch Optimierung der Linearfaktoren der Hauptkomponenten an die Situation angepaßt. In beiden Fällen erhält man schon ein relativ gutes Resultat, das durch Deformation noch an die Ränder und an die Gegenbezahnung angepaßt werden muss.

Gemäß Fig. 6. liefert eine Anpassung von Kauflächen je nach noch vorhandener Restzahnsubstanz fehlende Lücken im Bereich vor allem unterhalb des Zahnäquators. Diese Lücken müssen noch geschlossen werden. Obwohl die Auswahl von vollständigen Zahnoberflächen (d.h. inkl. Außenbereiche) möglich wäre, ist zur Zeit eine getrennte Anpassung von Kaufläche und Außenflächen (Oral-, Vestibulär, Approximalfläche) sinnvoll. Dadurch werden Parameter im Randbereich getrennt von Parametern im Kauflächenbereich behandelt und damit wird in den einzelnen Bereichen eine bessere Anpassung gewährleistet. Ausserdem ist der Vorgang der Ergänzung der Kauflächen, wie in der Erfindung erwähnt, automatisch durchführbar.

Gemäß Fig. 7 erhält man nach Anpassen an den Rand/ Gegenzahn und Ergänzung der fehlenden Flächen die gesamte Außenkontur (Außenhülle) des Zahnes. Wichtig ist dabei jeweils der glatte Übergang in den Randbereichen. Durch Zusammenfügen dieses Datensatzes an der Präparationsgrenze mit dem Datensatz der vermessenen Kavität/Defekt ist der gewünschte Formkörper für die CNC-Bearbeitung und Anfertigung in einer Produktionsmaschine aufbereitet.

Fig. 8.: Wenn keine oder nur wenig Restzahnsubstanz vorhanden ist (z.B. Kronenpräparationen), erfolgt die Ergänzung der fehlenden Außenflächen über den gesamten zirkulären Bereich. Dabei ist es sinnvoll, einige Konstruktionspunkte vorzugeben. Die Ergänzung wird in der Regel automatisiert ablaufen. Die weitere Anforderung ist ein glatter Übergang in den Randbereichen.

Fig. 9. zeigt ein Beispiel einer generisch erzeugten Zahnoberfläche. Hier handelt es sich z.B. um einen Durchschnittszahn, der aus 200 jugendlichen unversehrten ersten Oberkiefermolaren (OK-6er) berechnet wurde.

Fig. 10. und 11: Die generische Kaufläche mit den Hauptkomponenten kann wiederum an die Restgebißsituation angepaßt werden durch Verwendung der Restzahnsubstanz (Fig. 10) und/oder durch Auswahl bestimmter Punkte auf Bissregistrat (Fig.11) und/oder Nachbarzähne etc.. Im Gegensatz zur direkten Verwendung einer Zahnbibliothek kann mittels des generischen Zahnmodelldatensatzes die Auswahl von bestimmten Kontakt- und Merkmalspunkten bzw. -strukturen direkt vor der Berechnung und Konstruktion erfolgen, da es genügt, diese Punkte am generischen Zahn zu markieren. In der Zahnbibliothek müßte dagegen jeder einzelne Zahn mit den neuen Merkmalspunkten versehen werden. Dies erlaubt daher auch einen schnellen Wechsel je nach Situation, um verschiedene Okklusions- und Formkonzepte umzusetzen.

**Patentansprüche**

1. Verfahren zur Herstellung eines dreidimensionalen elektronischen Datensatzes eines generischen Zahnmodells unter Verwendung eines elektronischen Datensatzes eines für die Anfertigung eines Zahnersatzteils, einer Zahnrestauration oder eines Zahnmodells verwendbaren Durchschnittszahns, mit folgenden Verfahrensschritten:

   a) durch Vermessen einer vorbestimmten Mindestanzahl von Zähnen gleichen Zahntyps wird eine Vielzahl von elektronischen Datensätzen dieses Zahntyps erzeugt;
   b) es erfolgt eine Zuordnung zumindest einer Anzahl von für diesen Zahntyp charakteristischen Korrespondenzpunkten und/oder Korrespondenzstrukturen in den einzelnen elektronischen Datensätzen;
   c) unter Berücksichtigung der Zuordnung der Korrespondenzpunkte und/oder Korrespondenzstrukturen in den einzelnen Datensätzen erfolgt eine Mittelwertbildung aus den elektronischen Datensätzen;
   d) ein sich aus der Mittelwertbildung ergebender elektronischer Durchschnittsdatensatz wird als elektronische Darstellung eines Durchschnittszahns mit einer bezüglich der vermessenen Zähne durchschnittlichen Zahnoberfläche zur Verfügung gestellt,

   wobei nach erfolgter Zuordnung der Korrespondenzpunkte und/oder Korrespondenzstrukturen der Durchschnittsdatensatz von allen vermessenen Zahndatensätzen subtrahiert wird, anschließend eine Hauptachsenanalyse für die Differenzdatensätze durchgeführt wird, eine Linearkombination aus mindestens einem Teil der sich ergebenden Hauptachsen für den betrachteten Zahntyp gebildet wird und diese Linearkombination zusammen mit dem Durchschnittsdatensatz als generischer Zahnmodelldatensatz zur Verfügung gestellt wird.

2. Verfahren nach Anspruch 1, bei welchem die Zuordnung der Korrespondenzpunkte und/oder Korrespondenzstrukturen automatisch erfolgt.

3. Verfahren nach einem der Ansprüche 1 bis 2, bei welchem für die Zuordnung der Korrespondenzpunkte und/oder Korrespondenzstrukturen eine gewichtete Kombination zumindest aus Höhenwerten und Steigungen und/oder Krümmungen der entsprechenden elektronischen Daten verwendet wird.

4. Verwendung einer mit dem Verfahren nach einem der Ansprüche 1 bis 3 erhaltenen elektronischen Darstellung eines generischen Zahnmodells als elektronische Vorlage für eine Herstellung von physischen Zahnmodellen, Zahnrestaurationen oder Zahnersatzteilen mittels einer entsprechend dem generischen Zahnmodelldatensatz gesteuerten Maschine.

5. Verfahren zur Herstellung von physischen Zahnersatzteilen oder Zahnrestaurationen für reparaturbedürftige Zähne oder für Defektsituationen unter Verwendung einer mit dem Verfahren nach einem der Ansprüche 1 bis 4 erhaltenen elektronischen Darstellung eines generischen Zahnmodells, mit folgenden Schritten:

   a) es wird eine dreidimensionale Vermessung einer Präparation des reparaturbedürftigen Zahns oder einer Defektsituation durchgeführt und ein die Präparation oder Defektsituation darstellender elektronischer Datensatz erzeugt;
   b) aus der elektronischen Information der vermessenen Präparation oder der vermessenen Defektsituation werden für den Zahntyp des reparaturbedürftigen Zahns oder für den in die Defektsituation passenden Zahntyp charakteristische Korrespondenzpunkte und/oder Korrespondenzstrukturen ausgewählt;
   c) die Korrespondenzpunkte und/oder Korrespondenzstrukturen im elektronischen Datensatz der vermessenen Präparation oder Defektsituation werden entsprechenden Korrespondenzpunkten und/oder Korrespondenzstrukturen im Datensatz des generischen Zahnmodells zugeordnet;

d) die einander zugeordneten Korrespondenzpunkte und/oder Korrespondenzstrukturen werden durch ein Optimierungsverfahren möglichst weit gehend angenähert;

e) der durch die Optimierung ermittelte Datensatz wird einer Rekonstruktion des fehlenden Teils des reparaturbedürftigen Zahns oder zur Ergänzung der Defektsituation zu Grunde gelegt;

f) ein physisches Zahnersatzteil oder eine physische Zahnrestauration für den reparaturbedürftigen Zahn oder für die Defektsituation wird mittels einer Maschine hergestellt, die entsprechend dem bei dem Schritt e) erhaltenen Datensatz gesteuert wird.

6. Verfahren nach Anspruch 5 zur Herstellung eines dreidimensionalen elektronischen Datensatzes von Zahnersatzteilen oder Zahnrestaurationen, bei welchem nach erfolgter Zuordnung von Korrespondenzpunkten und/oder -strukturen des reparaturbedürftigen Zahnes und/oder der Defektsituation zum generischen Zahnmodelldatensatz die Linearfaktoren für den verwendeten Teil der Hauptachsen so optimiert werden, dass die neue Linearkombination möglichst gut die Korrespondenzen anpasst oder zur Deckung bringt.

7. Verfahren nach Anspruch 6, bei welchem die Linearfaktoren durch Minimierung der Abstände zwischen den Korrespondenzpunkten ermittelt werden

8. Verfahren nach Anspruch 6 oder 7, bei welchem die Linearfaktoren so ermittelt werden, dass die Wahrscheinlichkeit für die ermittelte Linearkombination möglichst hoch ist.

9. Verfahren nach einem der Ansprüche 5 bis 8, bei welchem die Optimierung die Wichtigkeit bestimmter Korrespondenzpunkte und/oder Korrespondenzstrukturen in Form von Wichtungsfaktoren berücksichtigt.

10. Verfahren nach einem der Ansprüche 5 bis 9, bei welchem elektronische Datensätze eines funktionellen Bissregistrats und/oder eines statischen Bissregistrats berücksichtigt werden.

11. Verfahren nach Anspruch 10, bei welchem die Information des Bissregistrats für die Bildung der Korrespondenzpunkte und/oder Korrespondenzstrukturen zur Rekonstruktion des reparaturbedürftigen Zahns und/oder der Defektsituation einbezogen wird.

12. Verfahren nach Anspruch 10 oder 11, bei welchem die möglichen Bereiche der Kontaktpunkte mit dem/die Gegenzahn/Gegenzähne als Korrespondenzpunkte und/oder Korrespondenzstrukturen ermittelt werden, indem durch Überlagerung des Datensatzes des statischen/okklusalen Bissregistrats mit dem Datensatz des funktionellen Bissregistrats die Bereiche mit geringen Abständen zwischen diesen Bissregistraten ausgewählt werden.

13. Verfahren nach einem der Ansprüche 5 bis 12, bei welchem von mindestens einem Nachbarzahn und/oder mindestens einem Gegenzahn und/oder mindestens einem symmetrisch gegenüberliegenden Zahn abgeleitete elektronische Daten für die Bildung der Korrespondenzpunkte und/oder Korrespondenzstrukturen zur Rekonstruktion des reparaturbedürftigen Zahns und/oder der Defektsituation einbezogen werden.

14. Verfahren nach einem der Ansprüche 1 bis 13, bei welchem der generische Zahnmodelldatensatz so bildlich dargestellt wird, dass die der vermessenen Präparation und/oder Defektsituation und/oder Bissregistrat/-e und/oder Nachbarzahn/Nachbarzähnen entsprechenden Korrespondenzpunkte und/oder Korrespondenzstrukturen direkt in eine elektronische graphische Darstellung des generischen Zahnmodelldatensatzes einzeichenbar sind.

15. Verfahren nach einem der Ansprüche 1 bis 14, bei welchem der generische Zahnmodelldatensatz zum Eingeben von Korrespondenzpunkten und/oder Korrespondenzstrukturen zusammen mit den vermessenen Datensätzen der Präparation und/oder Defektsituation und/oder Bissregistrat/-e und/oder Nachbarzahn/ Nachbarzähne bildlich dargestellt wird.

16. Verfahren zur Herstellung von Zahnrestaurationen unter Verwendung einer mit dem Verfahren nach einem der Ansprüche 1 bis 15 erhaltenen elektronische Darstellung eines generischen Zahnmodells, bei welchem die Einstellung der Abrasion der zu rekonstruierenden Zahnoberfläche zur Anpassung an den reparaturbedürftigen Zahn und/oder die Defektsituation und/oder Restgebisssituation aus dem generischen Zahnmodelldatensatz durch Variation der Linearfaktoren der Hauptkomponenten durchgeführt wird.

**17.** Verfahren nach Ansprüche 16, bei welchem die möglichen Bereiche der Kontaktpunkte mit dem oder die Gegenzähne als Korrespondenzpunkte und/oder Korrespondenzstrukturen ermittelt werden, indem durch Überlagerung des Datensatzes des statischen/okklusalen Bissregistrats mit dem Datensatz des funktionellen Bissregistrats des Gegenkiefers die Bereiche mit geringen Abständen zwischen diesen Bissregistraten ausgewählt werden.

**18.** Vorrichtung zur Veränderung eines mit einem Verfahren nach einem der Ansprüche 1 bis 4 erhaltenen generischen Zahnmodelldatensatzes, aufweisend eine Regeleinrichtung, mittels welcher die Linearfaktoren zumindest eines Teils der Hauptkomponenten des generischen Zahnmodelldatensatzes veränderbar sind und eine mit der Regeleinrichtung gekoppelten Darstellungseinrichtung zur bildlichen Darstellung des dem generischen Zahnmodelldatensatzes entsprechenden generischen Zahnes und der Auswirkung eines mittels der Regeleinrichtung durchgeführten Veränderung der Linearfaktoren.

**Claims**

**1.** Method for producing a three-dimensional electronic data set of a generic tooth model using an electronic data set of an average tooth that can be used for the preparation of a tooth replacement part, a tooth restoration or a tooth model, comprising the following method steps:

> a) a plurality of electronic data sets of a tooth type is generated by measuring a predetermined minimum number of teeth of the same tooth type;
> b) an assignment of at least one number of correspondence points and/or correspondence structures characteristic of this tooth type is carried out in the individual electronic data sets;
> c) averaging from the electronic data sets is carried out taking into account the assignment of the correspondence points and/or correspondence structures in the individual data sets;
> d) an electronic average data set resulting from the averaging is provided as an electronic illustration of an average tooth having a tooth surface that is average with respect to the measured teeth,

> wherein, after the assignment of the correspondence points and/or correspondence structures, the average data set is subtracted from all the measured tooth data sets, a principle axis analysis is carried out for the differential data sets, a linear combination of at least a portion of the resulting principle axes for the tooth type under consideration is formed and said linear combination is made available together with the average data set as a generic tooth model data set.

**2.** Method according to Claim 1, in which the assignment of the correspondence points and/or correspondence structures takes place automatically.

**3.** Method according to any of Claims 1 to 2, in which a weighted combination consisting at least of height values and slopes and/or curvatures of the corresponding electronic data is used for the assignment of the correspondence points and/or correspondence structures.

**4.** Use of an electronic illustration of a generic tooth model obtained with the method according to any of Claims 1 to 3 as an electronic template for a production of physical tooth models, tooth restorations or tooth replacement parts by means of a machine controlled in accordance with the generic tooth model data set.

**5.** Method for producing physical tooth replacement parts or tooth restorations for teeth in need of repair or for defect situations using an electronic illustration of a generic tooth model obtained with the method according to any of Claims 1 to 4, comprising the following steps:

> a) a three-dimensional measurement of a preparation of the tooth in need of repair or a defect situation is carried out, and an electronic data set representing the preparation or the defect situation is generated;
> b) characteristic correspondence points and/or correspondence structures for the tooth type of the tooth in need of repair or for the tooth type suitable for the defect situation are selected from the electronic information of the measured preparation or the measured defect situation;
> c) the correspondence points and/or correspondence structures in the electronic data set of the measured preparation or defect situation are assigned to corresponding correspondence points and/or correspondence structures in the data set of the generic tooth model;
> d) the correspondence points and/or correspondence structures that are assigned to one another are converged to the greatest extent possible using an optimization process;

e) the data set determined by the optimization is used as the basis for a reconstruction of the missing part of the tooth in need of repair or to amend the defect situation;

f) a physical tooth replacement part or a physical tooth restoration for the tooth in need of repair or for the defect situation is produced by means of a machine that is controlled in accordance with the data set obtained in Step e).

6. Method according to Claim 5 for producing a three-dimensional electronic data set of tooth replacement parts or tooth restorations, in which, after the assignment of correspondence points and/or structures of the tooth in need of repair and/or the defect situation to the generic tooth model data set, the linear factors for the used portion of the principle axes are optimized in such a way that the new linear combination fits or aligns the correspondences as well as possible.

7. Method according to Claim 6, in which the linear factors are determined by minimizing the distances between the correspondence points.

8. Method according to Claim 6 or 7, in which the linear factors are determined in such a way that the probability for the determined linear combination is as high as possible.

9. Method according to any of Claims 5 to 8, in which the optimization takes the relevance of specific correspondence points and/or correspondence structures into account in the form of weighting factors.

10. Method according to any of Claims 5 to 9, in which electronic data sets of a functional bite registration and/or a static bite registration are taken into account.

11. Method according to Claim 10, in which the information of the bite registration is included in the formation of the correspondence points and/or correspondence structures for the reconstruction of the tooth in need of repair and/or the defect situation.

12. Method according to Claim 10 or 11, in which the possible regions of the points of contact with the opposing tooth/opposing teeth are identified as correspondence points and/or correspondence structures and the regions with small distances between the bite registrations are selected by overlaying the data set of the static/occlusal bite registration with the data set of the functional bite registration.

13. Method according to any of Claims 5 to 12, in which electronic data derived from at least one adjacent tooth and/or at least one opposing tooth and/or at least one symmetrically opposite tooth is included in the formation of the correspondence points and/or correspondence structures for the reconstruction of the tooth in need of repair and/or the defect situation.

14. Method according to any of Claims 1 to 13, in which the generic tooth model data set is graphically displayed in such a way that the correspondence points and/or correspondence structures corresponding to the measured preparation and/or defect situation and/or bite registration(s) and/or adjacent tooth/adjacent teeth can be drawn directly into an electronic graphical display of the generic tooth model data set.

15. Method according to any of Claims 1 to 14, in which the generic tooth model data set is graphically displayed together with the measured data sets of the preparation and/or defect situation and/or bite registration(s) and/or adjacent tooth/adjacent teeth in order to input correspondence points and/or correspondence structures.

16. Method for producing tooth restorations using an electronic illustration of a generic tooth model obtained with the method according to any of Claims 1 to 15, in which the setting of the abrasion of the tooth surface to be reconstructed for adaptation to the tooth in need of repair and/or the defect situation and/or the situation of the rest of the teeth from the generic tooth model data set is carried out by varying the linear factors of the primary components.

17. Method according to Claim 16, in which the possible regions of the points of contact with the opposing tooth or opposing teeth are identified as correspondence points and/or correspondence structures and the regions with small distances between the bite registrations are selected by overlaying the data set of the static/occlusal bite registration with the data set of the functional bite registration of the opposing jaw.

18. Apparatus for modifying a generic tooth model data set obtained with a method according to any of Claims 1 to 4, comprising a control device, by means of which the linear factors of at least a portion of the primary components of the generic tooth model data set can be changed, and a display device coupled to the control device for graphically displaying the generic tooth corresponding to the generic tooth model data set and the effect of a modification of the linear factors carried out by means of the control device.

## Revendications

1. Procédé pour la production d'un ensemble de données électroniques tridimensionnel d'un modèle de dent générique en utilisant un ensemble de données électronique d'une dent moyenne pouvant être utilisée pour la fabrication d'une prothèse dentaire, d'une restauration de dents ou d'un modèle de dent, comprenant les étapes de procédé suivantes :

   a) une pluralité d'ensembles de données électroniques de ce type de dent sont générés par la mesure d'un nombre minimum prédéfini de dents du même type de dent ;
   b) une association d'au moins un certain nombre de points de correspondance et/ou de structures de correspondance caractéristiques dans les ensembles de données électroniques individuels est effectuée ;
   c) un calcul de moyennes des ensembles de données électronique est réalisé en tenant compte de l'association des points de correspondance et/ou structures de correspondance dans les ensembles de données individuels ;
   d) un ensemble de données moyennes électronique résultant du calcul de moyennes est mis à disposition comme représentation électronique d'une dent moyenne comprenant une surface de dent moyenne par rapport aux dents mesurées,

   l'ensemble de données moyennes étant soustrait de tous les ensembles de dents mesurés une fois que l'association des points de correspondance et/ou des structures de correspondance est effectuée, une analyse d'axes principaux étant ensuite effectuée pour les ensembles de données de différence, une combinaison linéaire d'au moins une partie des axes principaux résultants pour le type de dent considéré et cette combinaison linéaire étant mise à disposition ensemble avec l'ensemble de données moyennes comme ensemble de données de modèle de dent générique.

2. Procédé selon la revendication 1, dans lequel l'association des points de correspondance et/ou des structures de correspondance s'effectue automatiquement.

3. Procédé selon l'une quelconque des revendications 1 à 2, dans lequel une combinaison pondérée d'au moins des valeurs de hauteur et des pentes et/ou courbes des données électroniques correspondantes est utilisée pour l'association des points de correspondance et/ou des structures de correspondance.

4. Utilisation d'une représentation électronique d'un modèle de dent générique obtenue au moyen du procédé selon l'une quelconque des revendications 1 à 3 comme modèle électronique pour la fabrication de modèles physiques de dents, de restaurations de dent ou de prothèses dentaires au moyen d'une machine commandée selon l'ensemble de données de modèle de dent générique.

5. Procédé pour la fabrication de prothèses dentaires physiques ou de restaurations de dents pour des dents nécessitant une réparation ou pour des situations lésionnelles en utilisant une représentation électronique d'un modèle de dent générique obtenue avec le procédé selon l'une quelconque des revendications 1 à 4, comprenant les étapes suivantes :

   a) une mesure tridimensionnelle d'une préparation de la dent nécessitant réparation ou d'une situation lésionnelle est effectuée et un ensemble de données électronique représentant la préparation ou de la situation lésionnelle est généré ;
   b) des points de correspondance et/ou des structures de correspondance caractéristiques pour le type de dent de la dent nécessitant réparation ou du type de dent approprié pour la situation lésionnelle sont sélectionnés à partir des informations électroniques de la préparation mesurée ou de la situation lésionnelle mesurée ;
   c) les points de correspondance et/ou les structures de correspondance dans l'ensemble de données électronique de la préparation ou situation lésionnelle mesurée sont associés à des points de correspondance et/ou des structures de correspondance correspondants dans l'ensemble de données du modèle de dent générique ;
   d) les points de correspondance et/ou les structures de correspondance associés entre eux sont rapprochés le plus possible par un procédé d'optimisation ;

e) l'ensemble de données déterminé par l'optimisation est pris comme base pour une reconstruction de la partie manquante de la dent nécessitant réparation ou pour le complément de la situation lésionnelle ;

f) une prothèse dentaire physique ou une restauration de dent physique pour la dent nécessitant réparation ou pour la situation lésionnelle est fabriquée au moyen d'une machine qui est commandée conformément à l'ensemble de données obtenu dans l'étape e).

6. Procédé selon la revendication 5 pour la fabrication d'un ensemble de données électronique tridimensionnel de prothèses dentaires ou de restaurations de dents, dans lequel, après la réalisation de l'association de points et/ou structures de correspondance de la dent nécessitant réparation ou de la situation lésionnelle à l'ensemble de données de modèle de dent générique, les facteurs linéaires pour la partie utilisée des axes principaux sont optimisés de telle façon que la nouvelle combinaison linéaire adapte ou rapproche au mieux les correspondances.

7. Procédé selon la revendication 6, dans lequel les facteurs linéaires sont déterminés par la minimisation des distances entre les points de correspondance.

8. Procédé selon la revendication 6 ou 7, dans lequel les facteurs linéaires sont déterminés de telle façon que la probabilité pour la combinaison linéaire déterminée soit le plus élevé possible.

9. Procédé selon l'une quelconque des revendications 5 à 8, dans lequel l'optimisation tient compte de l'importance de certains points de correspondance et/ou structures de correspondance sous la forme de facteurs de pondération.

10. Procédé selon l'une quelconque des revendications 5 à 9, dans lequel des ensembles de données électroniques d'un enregistrement d'occlusion fonctionnel et/ou un enregistrement d'occlusion statique sont pris en compte.

11. Procédé selon la revendication 10, dans lequel les informations de l'enregistrement d'occlusion sont incluses pour la formation des points de correspondance et/ou des structures de correspondance pour la reconstruction de la dent nécessitant réparation et/ou de la situation lésionnelle.

12. Procédé selon la revendication 10 ou 11, dans lequel les zones potentielles des points de contacts avec le(s) dent(s) antagoniste(s) sont déterminées sous la forme de points de correspondances et/ou structures de correspondance, en sélectionnant, par la superposition de l'ensemble de données de l'enregistrement d'occlusion statique et de l'ensemble de données de l'enregistrement d'occlusion fonctionnel, les zones avec les distances les plus faibles entre ces deux enregistrements d'occlusion.

13. Procédé selon l'une quelconque des revendications 5 à 12, dans lequel des données découlées d'au moins une dent adjacente et/ou d'au moins une contre-dent et/ou d'au moins une dent symétriquement opposée sont incluses pour la formation des points de correspondance et/ou des structures de correspondance de la dent nécessitant réparation et/ou de la situation lésionnelle.

14. Procédé selon l'une quelconque des revendications 1 à 13, dans lequel l'ensemble de données de modèle de dent générique est représenté visuellement de telle façon que les points de correspondance et/ou structures de correspondance correspondant à la préparation et/ou situation lésionnelle et/ou enregistrement(s) d'occlusion et/ou dent(s) adjacente(s) mesurés peuvent être marqués directement dans la représentation graphique électronique de l'ensemble de données de modèle de dent générique.

15. Procédé selon l'une quelconque des revendications 1 à 14, dans lequel l'ensemble de données de modèle de dent générique est représenté visuellement avec les ensembles de données mesurés de la préparation et/ou de la situation lésionnelle et/ou du(des) enregistrement(s) d'occlusion et/ou de la(des) dent(s) adjacente(s) pour la saisie de points de correspondance et/ou de structures de correspondance.

16. Procédé pour la fabrication de restaurations de dents en utilisant une représentation électronique d'un modèle de dent générique obtenue avec le procédé selon l'une quelconque des revendications 1 à 15, dans lequel le réglage de l'abrasion de la surface de dent à reconstruire pour l'adaptation à la dent nécessitant réparation et/ou à la situation lésionnelle et/ou à la situation de dentition restante à partir de l'ensemble de données de modèle de dent générique est effectué par la variation des facteurs linéaires des composants principaux.

17. Procédé selon la revendication 16, dans lequel les zones potentielles des points de contacts avec le(s) dent(s) antagoniste(s) sont déterminées sous la forme de points de correspondances et/ou structures de correspondance, en sélectionnant, par la superposition de l'ensemble de données de l'enregistrement d'occlusion statique et de l'ensemble de données de l'enregistrement d'occlusion fonctionnel de la mâchoire opposée, les zones avec les distances les plus faibles entre ces deux enregistrements d'occlusion.

18. Dispositif pour la modification d'un ensemble de données de modèle de dent générique obtenu avec un procédé selon l'une quelconque des revendications 1 à 4, comprenant un dispositif de réglage, au moyen duquel les facteurs linéaires d'au moins une partie des composants principaux de l'ensemble de données de modèle de dent générique peuvent être modifiés, et un dispositif de présentation couplé au dispositif de réglage pour la présentation visuelle de la dent générique correspondant à l'ensemble de données de modèle de dent générique et de l'effet d'une modification des facteurs linéaires effectuée au moyen du dispositif de réglage.

Fig. 1

Fig. 2

Fig. 3

Fig. 4

z.B. Korrespondenzpunkte

Fig. 5

Fig. 6

Fig. 7

Begrenzungslinie
der Kaufläche

Präparationslinie

Interpolationspunkte

Fig. 8

*Fig. 9*

*Fig. 10*

*Fig. 11*

z.B. Kontaktpunkte zum Antagonisten

Fig. 12

Fig. 13

Fig. 14

Fig. 15

Fig. 16

Fig. 17

Fig. 18

Elektronische Datensätze von
vermessenen Zähnen
(Bibliothekszähne)
(geordnet z.B. nach Zahntyp)

Zuordnung der Korrespondenzen
(Korrespondenzpunkte,
Korrespondenzstrukturen) zwischen
den einzelnen Datensätzen
(z.B. automatisch für Meßpunkte,
Strukturen oder interaktiv)

Zuordnung der Korrespondenzen
(Korrespondenzpunkte,
Korrespondenzstrukturen) zwischen
den einzelnen Datensätzen
(z.B. automatisch für Meßpunkte,
Strukturen oder interaktiv)

Mittelwertbildung zwischen den
einzelnen Koordinaten der
jeweiligen Korrespondenzen liefert
den Durchschnittszahn der
betrachteten Datensätze

Hauptachsenanalyse der
Zahndatensätze. Diese liefert
Hauptkomponenten und Varianzen.

Differenz zwischen jeden einzelnen
elektronischen Datensatz und dem
Durchschnittszahn entsprechend
der Korrespondenzen

Linearkombination zumindest der
wichtigsten Hauptkomponenten
(die mit hohen Varianzanteilen)

Hauptachsenanalyse der
Differenzdatensätze. Diese liefert
Hauptkomponenten und Varianzen

Linearkombination zumindest der
wichtigsten Hauptkomponenten
(die mit hohen Varianzanteilen)
und Addition des
Durchschnittszahns.

**Generisches Zahnmodell**

**Durchschnittszahn**

Zurverfügungstellung als Zahnmodell
für Prothesenzähne, Übungsmodelle,
Internetapplikation,
Anschauungsmodelle,
Untersuchungsmodelle,
Druckerzeugnisse, Holographische
Bilder, Aufwachsmodell etc.

Zurverfügungstellung für die
Rekonstruktion und Herstellung
von Zahnersatzteilen und
Zahnrestaurationen

Fig. 19

## Rekonstruktion der Außenhülle

Dreidimensionale Vermessung der Präparation oder der Defektsituation. Elektronische Datensätze der Präparation bzw. Defektsituation.

Evtl. Einbeziehung der vermessenen Datensätze der Gegenkiefersituation (Antagonisten) z.B. in Form von okklusalen/statischen Bißregistrat, funktionellen Bißregistrat oder Gegenkiefermodell

Evtl. Einbeziehung der vermessenen Datensätze des/der Nachbarzähne

Bestimmung der Korrespondenzpunkte/strukuren mit dem generischen Zahnmodell, dem Durchschnittszahn oder den Bibliothekszähnen

Interaktive online-Bestimmung von Korrespondenzen während des Rekonstruktionsprozesses durch Auswahl von Punkten/ Strukturen in den dargestellten elektronischen Bildern von Vermessung und Durchschnittszahn bzw. generischem Zahnmodell

Auswahl derjenigen Korrespondenzpunkte/strukturen, die bereits für das generische Zahnmodell, den Durchschnittszahn oder die Bibliothekszähne hinterlegt wurden.

Automatische Korrespondenzzuordnung z.B. mittels optischen Fluß oder Matchingverfahren von Restzahnsubstanz zu Durchschnittszahn, generischem Zahnmodell oder Bibliothekszähnen

Zahnbibliothek

Generisches Zahnmodell

Durchschnittszahn oder Modellzahn

Ausgangspunkt für die Rekonstruktion von reparaturbedürftigen Zähnen oder Defektsituationen zur Herstellung von Zahnersatzteilen oder Zahnrestaurationen

Fig. 20

## Rekonstruktion der Außenhülle

| Generisches Zahnmodell | Durchschnittszahn oder Modellzahn | Zahnbibliothek |
|---|---|---|

| Möglichst optimierte Zuordnung der Korrespondenzpunkte /strukturen zwischen vermessenen Datensätzen und generischem Zahnmodell | Möglichst optimierte Zuordnung der Korrespondenzpunkte /strukturen zwischen vermessenen Datensätzen und Durchschnittszahn | Möglichst optimierte Zuordnung der Korrespondenzpunkte /strukturen durch Auswahl des bestpassendsten Zahns aus der Bibliothek |
|---|---|---|

| Bestimmung der Linearfaktoren für die für das generische Zahnmodell verwendeten Hauptkomponenten so, dass 'ne Fehlerfunktion (z.B. Abstandsfunktion) minimiert wird (die Parameter der Translation, Rotation und Skalierung und allgemein affine Transformationen können zusätzlich in die Minimierung mit einbezogen werden). | Bestimmung der Rotations- ,Translations- und/oder Skalierungsparameter bzw. Parameter einer affinen Transformation durch Minimierung einer Fehlerfunktion | Bestimmung der Linearfaktoren für die für das generische Zahnmodell verwendeten Hauptkomponenten so, dass die Wahrscheinlichkeit für den neugebildeten Zahn möglichst maximal wird. (die Parameter der Translation, Rotation , Skalierung und/oder allgemein affine Transformationen können zusätzlich in die Minimierung mit einbezogen werden) |
|---|---|---|

Evtl. Anpassung des ermittelten Datensatzes durch Deformation, Mörphing etc. in den Bereichen, in denen sich noch Störstellen oder Problemzonen befinden (Kontaktpunkte nicht an der richtigen Stelle, Durchdringung des funktionellen oder statischen Bissregistrates, Übergang zur Präparatgrenze, glatte Anpassung an die Restzahnsubstanz, Ausschneiden entsprechend dem Defekt, Schichtstärke zu gering etc)

Ergänzung noch evtl. fehlender Aussenflächenteile (z.B. durch glatte Anbindung von Spline-, Bezier-, NURBS-Flächen etc.). Durch Verwendung von Kontrollpunkten können diese Flächen noch exakter geformt werden

Datensatz der rekonstruierten Aussenhülle für den reparaturbedürftigen Zahn und/oder Defektsituation

Fig. 21

## Anfertigung eines Zahnersatzteils oder einer Zahnrestauration

```
┌─────────────────────────────┐
│ Datensatz der rekonstruierten │
│ Aussenhülle für den          │
│ reparaturbedürftigen Zahn    │
│ und/oder die Defektsituation │
└─────────────────────────────┘
              │
              ▼
┌─────────────────────────────┐
│ Verknüpfung des Datensatzes der │
│ Präparation entlang der      │
│ Präparationsgrenze mit dem Datensatz │
│ der rekonstruierten Außenhülle │
└─────────────────────────────┘
              │
              ▼
┌─────────────────────────────┐
│ Evtl. Anpassung des Datensatzes an die │
│ Werkzeuggeometrien und       │
│ Fertigungsbedingungen einer für die │
│ Anfertigung verwendeten Maschine │
└─────────────────────────────┘
              │
              ▼
┌─────────────────────────────┐
│ Steuerung der Maschine mit dem │
│ Datensatz und Anfertigung der │
│ Zahnrestauration bzw. des    │
│ Zahnersatzteils              │
└─────────────────────────────┘
```

Fig. 22

## Anfertigung eines Zahnmodells

```
┌─────────────────────────┐      ┌─────────────────────────┐
│ Generisches Zahnmodell  │      │ Durchschnittszahn       │
└─────────────────────────┘      └─────────────────────────┘
              ╲                    ╱
               ╲                  ╱
                ▼                ▼
┌─────────────────────────────────────┐
│ Evtl. Deformationen und/oder Morphing, │
│ evtl. Anpassung des Datensatzes an die │
│ Werkzeuggeometrien und              │
│ Fertigungsbedingungen einer für die │
│ Anfertigung verwendeten Maschine    │
└─────────────────────────────────────┘
              │
              ▼
┌─────────────────────────────┐
│ Steuerung der Maschine mit dem │
│ Datensatz und Anfertigung des │
│ Zahnmodells                  │
└─────────────────────────────┘
```

Fig. 23

**EP 3 091 454 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5217375 A
- EP 0643948 A
- EP 0634150 A
- EP 0913130 A2
- WO 0239056 A
- EP 0643948 A1
- DE 19838239 A1
- DE 19923978 A1
- US 5257203 A
- US 20020015934 A1
- DE 19642247

**In der Beschreibung aufgeführte Nicht-Patentliteratur**

- **MATTIOLA, A. ; MÖRMANN, W.H. ; LUTZ, F.** Computerunterstützte Okklusion von Cerec 2 Inlays und Overlays. *Schweiz Monatsschr Zahnmed,* 1995, vol. 105, 1283-1290
- **KUNZELMANN, K.-H. ; MEHL, A. ; PELKA, M.** Automatische Rekonstruktion von Kauflächen computergenerierter Restaurationen. *Zahnärztl Welt/Rundschau,* 1993, vol. 102, 695-703
- **MEHL, A. ; GLOGER, W. ; HICKEL, R.** Erzeugung von CAD-Datensätzen für Inlays und Kronen mit funktionellen Kauflächen. *Dtsch Zahnärztl Z,* 1997, vol. 52, 520-524
- In Cerec 10 year anniversary Smposium. **SALIGER, G.** Designing a CEREC crown. Quintessence, 1996
- 3D Correspondence. **SHELTON, C.R.** Master Thesis. Massachusetts Institute of Technologie, 1998
- **UMEYAMA S.** Least-squares estimation of transformation parameters between two point patterns. *IEEE PAMI,* 1991, vol. 13 (4), 276-280
- **BLANZ, V. ; ROMDHANI, S.** Face Identification across different poses and illuminations with a 3D morphable model. *Proc. Int. Conference on Automatic Face and Gesture Recognition,* 2002, 202-207